(19) Europäisches Patentamt
European Patent Office
Office européen des brevets

(11) EP 4 225 388 B1

(12) EUROPEAN PATENT SPECIFICATION

(45) Date of publication and mention of the grant of the patent:
**09.09.2026 Bulletin 2026/37**

(21) Application number: **21798089.5**

(22) Date of filing: **08.10.2021**

(51) International Patent Classification (IPC):
***A61L 27/18*** $^{(2006.01)}$

(52) Cooperative Patent Classification (CPC):
(C-Sets available)
**A61L 27/18;** A61L 2430/04 (Cont.)

(86) International application number:
**PCT/GB2021/052614**

(87) International publication number:
**WO 2022/074401 (14.04.2022 Gazette 2022/15)**

(54) **IMPLANT**

IMPLANTAT

IMPLANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.10.2020 GB 202016076**

(43) Date of publication of application:
**16.08.2023 Bulletin 2023/33**

(73) Proprietor: **4D Medicine Limited (T/A 4D Biomaterials)**
**Birmingham, West Midlands B15 2SQ (GB)**

(72) Inventors:
- **NAYLOR, Andrew**
**Edgbaston,**
**Birmingham West Midlands B15 2SQ (GB)**
- **DOVE, Andrew Peter**
**Edgbaston,**
**Birmingham West Midlands B15 2SQ (GB)**
- **SMITH, Philip Norman**
**Edgbaston,**
**Birmingham West Midlands B15 2SQ (GB)**

(74) Representative: **HGF**
**HGF Limited**
**4th Floor, 1 City Square**
**Leeds LS1 2ES (GB)**

(56) References cited:
**US-A1- 2019 038 812**

- **JUN FENG ET AL: "Construction of functional aliphatic polycarbonates for biomedical applications", PROGRESS IN POLYMER SCIENCE, PERGAMON PRESS, OXFORD, GB, vol. 37, no. 2, 22 July 2011 (2011-07-22), pages 211 - 236, XP028395656, ISSN: 0079-6700, [retrieved on 20110802], DOI: 10.1016/J.PROGPOLYMSCI.2011.07.008**
- **ROKICKI ET AL: "Aliphatic cyclic carbonates and spiroorthocarbonates as monomers", PROGRESS IN POLYMER SCIENCE, PERGAMON PRESS, OXFORD, GB, vol. 25, no. 2, 1 March 2000 (2000-03-01), pages 259 - 342, XP027184957, ISSN: 0079-6700, [retrieved on 20000301]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 27/18, C08L 75/04**

## Description

The present invention relates to an implant, in particular a void occlusion device for implanting into a void in a body tissue. This invention further relates to a kit comprising the implant, methods of manufacturing and using the implant.

Surgical treatments including biopsies and the removal of tumours or neoplasms often leaves a void in the tissue. Initially the void fills with fluid in response to the injury/surgery. However, over time the fluid is reabsorbed, and the resulting cavity collapses due to lack of structural support. These procedures frequently lead to dimpling and other disfigurements unless a prostheses or implant is deployed within the cavity from which tissue has been removed.

A prime example of this is lumpectomies, where a tumour is removed from the breast. A surgically closed lumpectomy-cavity may fill with fluid, sustaining the breast's shape postoperatively. As much of the breast as possible is conserved. However, the surgery can significantly change the breast's shape as, once fluid drains from the void, it collapses and the breast dimples or deflates, impacting shape (which may result in asymmetry with the opposing breast), causing pain and/or preventing healing. The location of tissue removal and the pre-existing breast size often impact the aesthetic deformity that ensues. Little can often be done to restore the normal breast contour once this process is completed, the resulting deformity is essentially permanent.

Further, breast deformation due to lumpectomy also complicates precise targeting of the tissue in patients who require post-operative radiotherapy, increasing the patient's risk of secondary cancer.

The potential breast deformity (size discrepancy) following lumpectomy is a principal determinant affecting the selection of surgical treatment for breast tumour removal, particularly in younger patients (less than 45 years of age).

The original treatment for removal of breast tumours is a mastectomy (complete removal of the breast). Mastectomies are a riskier, more invasive surgery that can cause extreme aesthetic/cosmetic changes to the patient and require follow-up surgical procedures to remake the breast. Further, the procedure may not only be physiologically but also psychologically traumatic to a patient. However, mastectomies are often favoured, particularly by clinicians who lack confidence in contemporary lumpectomy treatment and are concerned about precise post-surgical radiotherapy targeting following lumpectomies.

Treatment options to overcome the deformity left by a lumpectomy or partial mastectomy include oncoplastic surgery which involves immediate reconstruction of the breast. The goal of oncoplastic surgery is to reshape the breasts to minimise the effects of surgery, which can help a patient recover and heal both physically and emotionally. However, oncoplastic surgery may involve operating on both breasts, including one that may not have cancer, to make the two breasts symmetrical, resulting in additional scarring and increased risk. The oncoplastic approach to treat breast deformity is therefore largely impractical and requires a surgeon to have adequate training or requires coordination with a plastic surgeon.

New treatments seek to utilise implants such as tissue marking and void occlusion devices. However, these devices are aimed at enhancing radiotherapy imaging and suffer from poor cosmetic restoration and healing.

Primary 3D printing material focus has remained on acrylate- and epoxide-containing polymers. Residual groups and unreacted monomers present in these polymers, which can leach out, are highly toxic. The primary degradable biomaterial focus has been directed towards poly(L-lactic acid) (PLLA), which is limited by its poor processability in photo polymerizations and its acidic degradation products.

Biozorb® is a 3D implantable marker that consist of a spiral, framework embedded with six permanent, titanium clips designed to precisely mark the surgical excision site. The implantable marker, which is designed to absorb into the body over several years, provides more precise targeting, reducing radiotherapy costs. However, the device is often combined with oncoplastic reconstruction (reconstructive lumpectomy) as it does not deal with the preservation of breast shape. Alongside lack of cosmetic benefit, further limitations include: painful swelling, skin discoloration and irritation at the implant site, poor healing and tissue response to the implant over time, poor resorption by the body and difficulty to fit in patients, as the device cannot be modified by the clinician. More serious problems include allergic reactions, device failure and necrosis at the implant site.

US 2019/038812 A1 discloses a soft tissue filler for application at a lumpectomy site comprising an aminoacid derived biodegradable polycarbonate-urethane scaffold having a porosity of between about 80% and about 95%, a compressive moduli of between about 1 kPa and about 50 kPa, a swelling capacity of between about 100% and about 300%, and a dry volume of 50 $mm^3 \pm 25$ $mm^3$.

The major factors that are considered when developing new biomaterials are: (i) clinical requirements such as mechanical strength and biocompatibility, (ii) fabrication of the biomaterials for optimum implant design and (iii) cost requirements. Existing polyester-based materials that are widely used for medical implants have a number of limitations, including: acidic and inflammatory degradation products; brittle, limited mechanical behaviour; low absorption rates; difficult, expensive to manufacture; swelling when implanted; accelerated degradation of strength; impingement of nerves and vessels which can cause pain and other issues; and limited storage and shelf life of polymeric products.

It is therefore a non-exclusive object of the invention to provide a device that overcomes one or more drawbacks of the prior art.

Summary of invention

The scope of the invention is defined by the claims. Any references in the description to methods of treatment refer to the products of the present invention for use in a method for treatment.

Accordingly, a first aspect of the invention provides an implant for inserting into a void in a body tissue in accordance with Claim 1.

The implant may be for implanting into a void in tissue. Voids in tissue may be due to a deformity, or they may be caused by trauma or surgery, for example removal of a tumour. The implant may therefore be described as a void occlusion device.

The tissue may be soft tissue, such as fat, muscle or fibrous tissue. In some embodiments, the implant is for use in occlusion of a void in a breast of a subject following a lumpectomy procedure. Thus, the implant may be a post-lumpectomy implant. In some embodiments, the implant is for occlusion of a void in hard tissue, such as bone.

In use, the implant may be capable of assuming the size and shape of the void in the second expanded state. Surprisingly, the inventors have discovered that the implant is able to expand to fill a void without putting undue pressure on the surrounding material (i.e. tissue), even if the void is irregular in shape. Furthermore, it has been found that the polymeric material is able to expand to fill the void without requiring personalisation of the implant prior to insertion. Thus, the implant deforms to the shape of the void and becomes locked in position. These properties are particularly beneficial for void occlusion, since it enables the whole void to be filled by the implant, thereby supporting the surrounding tissue, preventing its collapse into the void and promoting healing of the entire void, without causing damage to the surrounding tissue or pain. Beneficially, this is achieved with a peak expansion rate as set out above.

In some embodiments, the implant is 3D printed.

The polymeric material comprises a crossed-linked polymer. For example, a crossed-linked polycarbonate, cross-linked poly(carbonate-co-urethane), cross-linked poly(carbonate-co-urea) or cross-linked poly(carbonate-co-amide).

The polymeric material is formed from a resin composition comprising a prepolymer and optionally one or more diluents, wherein the prepolymer comprises repeating units having at least one carbonate linkage. Either or both of the prepolymer and the at least one optional diluent(s) may comprise at least one O=C-N linkage, preferably a urethane linkage.

The prepolymer is poly(TMPAC), poly(NTC) or poly(TMPAC-co-NTC). The ratio of TMPAC (5-[(allyloxy) methyl]-5-ethyl-1,3-dioxan-2-one) to NTC (9-(5-norbornen-2-yl)-2,4,8,10-tetraoxa-3-spiro[5.5]undecanone) monomers in the prepolymer may be from 100:0 to 0:100, from 90:10 to 10:90, from 80:20 to 20:80, from 75:25 to 25:75, or from 60:40 to 40:60.

Preferably the implant is biocompatible. In some embodiments, the implant is bioresorbable.

The implant may have an *in vivo* life of at least 4 weeks. In some embodiments, the implant has an *in vivo* life of no more than 5 years, no more than 4 years, no more than 36 months, no more than 30 months or no more than 24 months.

In some embodiments, the polymeric material comprises an imaging agent, optionally wherein the imaging agent comprises a radiopaque material.

In some embodiments, the polymeric material comprises a biologically active agent, optionally wherein the biologically active agent is selected from an antimicrobial, an anti-inflammatory agent or an anti-cancer agent.

The implant may be in the form of a mesh having a pore size of from 50 to 2000 μm, from 100 to 1800 μm, from 200 to 1500 μm, from 300 to 1200 μm, from 400 to 1000 μm, from 500 μm to 800 μm or from 600 to 700 μm.

According to a second aspect of the invention there is provided a kit for reconstruction of tissue following a surgical procedure, the kit comprising at least one implant according to the first aspect of the invention, and instructions for use.

In some embodiments, the kit is for the reconstruction of soft tissue following surgery, for example for reconstruction of a breast following a lumpectomy procedure. In some embodiments the kit is for repairing hard tissue, such as bone, following trauma or surgery.

The kit may comprise at least two implants which differ from each other in at least their size, shape, material or mechanical properties.

In some embodiments, the kit comprises a first implant, a second implant and a third implant, wherein the second implant is greater in volume than the first implant, and the third implant is greater in volume the second implant.

The kit may further comprise at least one of:

- an instrument for inserting the implant into the void;
- apparatus for compressing the implant prior to insertion; and/or
- a stimulating device or reagent for causing the implant to transition from a compression to an expanded state.

According to a third aspect of the invention there is provided a method of manufacturing an implant for implanting

into a void in a body tissue in accordance with Claim 12.

The prepolymer may comprise repeating units having at least one carbonate linkage and at least one unsaturated side-chain, and the diluent may comprise at least one unsaturated side-chain, wherein either or both of the prepolymer and the diluent comprises at least one O=C-N linkage, preferably a urethane linkage.

In some embodiments, the steps of shaping the resin composition and cross-linking the prepolymer are carried out simultaneously, optioning by 3D printing (e.g. using stereolithography or microstereolithography).

The resin composition may have a viscosity of no more than 20 Pa.s, no more than 18 Pa.s or no more than 15 Pa.s at 22 °C.

The method may further comprise modifying the implant by turning, milling, sanding, filing, cutting, drilling and/or compressing the implant. Compress the implant may comprise:

a. heating the implant to a temperature greater than the glass transition temperature of the polymeric material;
b. compressing the implant; and
c. fixing the implant in the compressed form, optionally by cooling.

In some embodiments, the method further comprises determining the dimensions of the void, and manufacturing an implant having a desired size and shape based on the determined dimensions of the void.

The method may further comprise adding a biologically active agent and/or an imaging agent to the resin composition and/or to the polymeric material.

According to a fourth (unclaimed) aspect of the invention there is provided a method of reconstructing tissue having a void therein, the method comprising inserting a biocompatible implant according to the first aspect of the invention into the void.

The method may be for reconstructing tissue following a surgical procedure that results in a void in the tissue. For example, the surgical procedure may have removed a tumour in the tissue, e.g. a lumpectomy. Alternatively, the method may be for reconstructing tissue that is deformed, wounded or has been subjected to a trauma.

In some embodiments, the method comprises the implant in a compressed state and, after insertion, exposing the implant to a stimulus causing it to expand, thereby filling the void. The method may further comprise compressing the implant, prior to insertion.

In some embodiments, the method further comprises determining the dimensions of the void. The method may additionally comprise:

d. selecting an implant based on the determined dimensions of the void. For example, an implant may be selected which is approximately the same size as the void, or preferably larger than the void, in the expanded state;
e. providing an implant and modifying the size and/or shape of the implant according to the dimensions of the void; or
f. manufacturing an implant having a desired size and shape based on the determined dimensions of the void.

In some embodiments, the method further comprises suturing the implant into the void.

According to a fifth aspect of the invention, there is provided a method of identifying a target site for radiotherapy in a subject in need thereof, the method comprising determining the location of an implant as defined herein, optionally wherein the polymeric material comprises an imaging agent.

Embodiments of the invention will now be described by way of example only with reference to the accompanying drawings in which:

Figure 1 is an implant according to an embodiment of the invention;
Figure 2 a synthetic route to a prepolymer for use in a resin composition, according to an embodiment of the invention;
Figure 3A is a schematic reaction of iodination post polymerisation functionalisation of a polymer, according to an embodiment of the invention;
Figure 3B is a graph comparing the x-ray density of a non-iodinated polymer and an iodinated polymer, according to embodiments of the invention;
Figure 3C is a schematic reaction showing alkylation post-polymerisation functionalisation of a polymer, according to embodiments of the invention;
Figure 4 is a schematic approach to the treatment procedure using an implant of the invention, wherein the surgical procedure is a lumpectomy;
Figure 5 is an overview of the treatment options and outcomes for patients requiring surgery for breast cancer;
Figure 6A is an absorbance spectrum of the photoinitiator and photoinhibitors used in a resin composition, according to an embodiment of the invention;
Figures 6B and 6C are graphs showing the gelation times corresponding with photorheological phase transition behaviour studies of resins and monomers, according to embodiments of the invention;

Figure 6D is a graph showing the storage moduli for resins, according to embodiments of the invention, over time;
Figure 6E is a graph shown resin shrinkage over the course of film curing, according to an embodiment of the invention;
Figure 6F is a graph showing the rate of cross-linking over time, according to an embodiment of the invention;
Figure 6G is a graph showing viscosity vs diluent concentration, according to an embodiment of the invention;
Figure 6H is a graph of viscosity vs photoinitiator concentration, according to an embodiment of the invention;
Figure 6I is a schematic showing the digital light processing 3D printing process used to produce implants of the invention;
Figure 7A shows representative images of adipocytes and fibroblasts for PTMPTCX and PNTCTX scaffolds, according to embodiments of the invention;
Figure 7B shows confocal images of 3D PTMPTCX scaffolds after 7 days proliferation;
Figure 7C shows a representative printed stair-step pyramidal structure with corresponding cell images, displaying cell migration after 7 days;
Figure 7D shows representative images of cellular proliferation throughout PTMPTCTX foam;
Figures 8A to 8C are graphs showing the thermomechanical properties of polymeric materials of the invention, showing the relationship between $T_g$ and NTC concentration, stress-strain behaviour and cyclic-compression behaviour;
Figure 8D shows representative images of the PTMPTCX scaffold before loading, under strain and after loading is removed;
Figure 8E is a graph showing energy absorption for 100 cycles in alginate gels;
Figure 9 is a stress-strain recovery plot for compressed scaffolds immersed in 37 °C;
Figure 10A are representative images showing the shape memory behaviour of a printed polyNTC scaffold, according to embodiments of the invention;
Figure 10B shows the void filling of regular and irregular, hard and soft, voids with a polymeric material or implant formed therefrom, according to an embodiment of the invention;
Figure 10C is a graph showing the void filling efficiency and strain recovery of PTMPCTX and PNTCTX scaffolds of the invention;
Figures 10D and 10E are graphs showing the expansion forces of PTMPTCX and PNTCTX scaffolds, according to embodiments of the invention;
Figure 10F is an finite element analysis (FEA) plot determining simulated expansion force;
Figure 11A are representative microscopy images of printed PTMPTCX scaffolds, according to an embodiment of the invention, showing the surface erosion behaviour;
Figure 11B to 11G are graphs showing the swelling and degradation behaviour of 3D printed materials, according to embodiments of the invention;
Figure 12 shows representative histological images of PTMPTCX films, according to embodiments of the invention; and
Figures 13 A and 13B are graphs showing the strain recovery behaviors of printed scaffolds as a function of time and composition at 25 °C (Figure 13A) and 37 °C (Figure 13B).

Detailed description

**Implant**

In an aspect of the invention, there is provided an implant for implanting into a void in a body tissue. For example, the void may be caused by a wound or following a surgical procedure that results in a void in the tissue. In some embodiments, the implant is for filling voids in soft tissue. In some embodiments, the implant is for filling voids in hard tissue, such as bone. The implant of the invention may be considered to be a void occlusion device. The terms device and implant may be used interchangeably.
In a preferred embodiment, the implant is a post-lumpectomy implant.
The polymeric material or implant formed therefrom is capable of transitioning from a compressed state to an expanded state upon exposure to said stimulus.
The polymeric material may be a shape memory polymer. As used herein, a "shape memory polymer" is a polymer which can exist in a permanent state and a temporary state, the permanent state being capable of undergoing a morphological change to the temporary state, or vice versa, upon induction by an external stimulus. For example, the permanent state may be the state of the polymeric material or implant "as-formed", such as an expanded state. The temporary state may be a compressed form of the polymeric material or implant. Upon induction by the stimulus, the polymeric material or implant may revert from the temporary (e.g. compressed) state to its permanent (e.g. expanded) state. Thus, the polymeric material retains "memory" of its expanded, permanent state and is able to revert back to it under

certain conditions.

The external stimulus may be a temperature change, for example, heating or cooling, such as heating or cooling to approximately physiological temperature. The external stimulus may comprise one or more of direct or Joule heating, radiation and laser heating, microwaves, pressure, moisture (e.g. water), the presence or absence of solvent or solvent vapours, and/or change in pH. In some embodiments the external stimulus is a temperature change or moisture. Preferably, the external stimulus is heating (e.g. to physiological temperature) or water.

The implant may be capable of assuming the size and shape of the void in the second expanded state. In the expanded state, the size and shape of the implant may be complementary to the size and shape of the void in the body tissue. In the compressed state, the implant may adopt a compact, flexible and/or deployable shape. Such a shape may be beneficial for minimally invasive delivery to said void within a patient. Advantageously, the polymeric material enables void filling without personalisation of the implant structure, even in the case of irregularly-shaped voids.

It will be appreciated that a further external stimulus may be required to transform the polymeric material, or implant formed therefrom, from the expanded state to the compressed state. This further external stimulus may be different to the external stimulus which induces the transition from the compressed (e.g. temporary) state to the expanded (e.g. permanent) state. In some embodiments, compression of the polymeric material or implant formed therefrom is achieved by applying a force to the polymeric material or implant formed therefrom. Thus, in some embodiments the further external stimulus comprises a physical force to which the polymeric material or implant is subjected.

Preferably the implant is biocompatible. By "biocompatible", it will be understood that the polymeric material, and the implant formed therefrom, is not harmful or toxic to living tissue. The implant is therefore able to exist in the body without causing local or systemic deleterious effects, and without causing an immune response.

## Material of the implant

The polymeric material or an implant formed therefrom is formed from a resin composition. The resin composition comprises a prepolymer and optionally one or more diluent(s). For example, the resin composition may comprise polycarbonate oligomers (i.e. prepolymers), such as aliphatic polycarbonate oligomers.

The resin composition further comprises one or more crosslinkers. The resin composition further comprises one or more reactive diluents and/or chain extenders. These components enable the production of resins with tuneable viscosities.

As used herein, the term "prepolymer" refers to a polymerizable compound from which the polymeric material may be formed. The prepolymer may itself be a polymer. For example, the prepolymer may be an oligomer of a linear polycarbonate homopolymer comprising carbonate monomers.

In some embodiments, the prepolymer has a number-average molar mass ($M_n$) of no more than about 5 kDa, no more than about 4 kDa, no more than about 3 kDa, no more than about 2.5 kDa or no more than about 2 kDa. In some embodiments, the prepolymer has a number-average molar mass ($M_n$) of at least 1 kDa, at least 1.5 kDa, at least 2 kDa or at least 2.5 kDa.

The prepolymer comprises repeating units having at least one carbonate linkage and, optionally, at least one unsaturated side chain.

Either or both of the prepolymer and the at least one optional diluent(s) may comprise at least one O=C-N linkage, preferably a urethane linkage and/or a urea linkage. Advantageously, controlling the amount or number of urethane and/or urea linkages in the composition enables the shape memory behaviour of the polymer to be controlled.

In some embodiments, the repeating units of the prepolymer comprise at least one urethane linkage. In some embodiments the prepolymer is a polycarbonate, e.g. poly(carbonate-co-urethane). In some embodiments, the prepolymer is selected from poly(carbonate-co-urethane), poly(carbonate-co-urea), poly(carbonate-co-amide), poly(carbonate-co-thiourea).

It will be appreciated that the polymeric material comprises features of the prepolymer and, optionally the diluent(s), cross-linker(s) and/or chain extender(s) from which it is formed. Thus, in some embodiments the polymeric material comprises carbonate linkages. In some embodiments the polymeric material comprises O=C-N e.g. urethane linkages. In some embodiments, the polymeric material comprises cross-linked polycarbonate, cross-linked poly(carbonate-co-urethane), cross-linked poly(carbonate-co-urea), cross-linked poly(carbonate-co-amide), or cross-linked poly(carbonate-co-thiourea).

The prepolymer, and/or the or each optional diluent(s), may comprise at least one side-chain.

In some embodiments, the prepolymer comprises repeating units having at least one side chain. The side chains may be selected from: a n-alkyl chain, a branched alkyl chain, an alkyl chain comprising unsaturated moieties, an alkyl chain comprising heteroatoms (for example, fluorine, chlorine, bromine, iodine, oxygen, sulphur, nitrogen), or a combination thereof. The alkyl chain may comprise unsaturated portions, comprising alkenes, or aromatic moieties. The alkyl chain may be substituted by one or more functional groups (e.g. 1-5 or 2-3 functional groups). For example, the functional groups may be one or more of an azide, a carbonyl group, an alcohol, a halogen, a thiol or an alkene. Such functional groups may

conveniently be used to further derivatise the oligomers or the polymeric material formed therefrom.

In some embodiments, the prepolymer, and/or the or each optional diluent(s), comprises at least one unsaturated side chain, e.g. an alkyl chain comprising an unsaturated moiety.

The unsaturated side chains of the prepolymer and/or diluent(s) may be capable of being crosslinked. The polymeric material comprises a cross-linked polymer. Some unsaturated side chains may remain unreacted following polymerisation (i.e. cross-linking). Therefore, in some embodiments, the polymeric material may comprise unsaturated side chains. Unsaturated side chains present in the resin composition, or in the polymeric material formed therefrom, may be further functionalised to impart desired properties to the polymeric material. For example, the unsaturated side chains may be halogenated, e.g. iodinated.

In some embodiments the polymeric material comprises branched or unbranched alkyl side chains (e.g. $C_2$-$C_{10}$ alkyl chains) substituted by a halogen (e.g. fluoro, chloro, bromo or iodo) or thiol group. Preferably the halogen is an iodo group.

In some embodiments, the resin composition is photocurable. The resin composition may comprise at least one photoinitiator.

In some embodiments the resin composition comprises a prepolymer, a first photoinitiator, and a second distinct photoinitiator, the prepolymer comprising a repeating unit, the repeating unit comprising a first functional group and a distinct second functional group, the first photoinitiator having a first absorption wavelength to functionalise the first functional group, and the second distinct photoinitiator having a second absorption wavelength to functionalise the second functional group.

In some embodiments, the resin composition comprises at least one photoinhibitor. The photoinhibitor may be selected such that it absorbs light at approximately the same wavelength as the photoinitiator. A photoinhibitor having competitive absorbance in substantially the same region as the photoinitiator is advantageous because it provides spatial control by preventing light penetration beyond the layer that is being cured.

Prepolymers comprising repeating units containing at least one carbonate linkage may be generated using organocatalytic ring opening polymerization (ROP), as described herein. For example, homo- and co-oligocarbonate prepolymers may be formed from 6-membered cyclic carbonates, e.g. from allyl- and norbornene-containing monomers (TMPAC and NTC respectively). The prepolymer (i.e. oligomer) comprises or is constituted by poly(TMPAC), poly(NTC) or poly(TMPAC-co-NTC).

The components of the resin composition, and their relative amounts, may be modified in order to tune the properties of the polymeric material. For example, the ratios of the monomers used to prepare the prepolymers may be varied to impart different structural and functional properties to the polymeric material. In some embodiments, the ratio of TMPAC to NTC monomers in the prepolymer may be from 100:0 to 0:100, from 95:5 to 5:95, from 90:10 to 10:90, from 80:20 to 20:80, from 75:25 to 25:75, from 70:30 to 30:70, from 65:45 to 45:65, from 60:40 to 40:60 or from 55:45 to 45:55. In some embodiments, the ratio is 100:0, 75:25, 50:50, 25:75 or 0:100. In some embodiments, the ratio is 100 TMPAC : 0 NTC. Advantageously, 100% TMPAC forms a soft material with a modulus similar to that of soft tissue.

In an embodiment the resin composition may be selected such that the mechanical properties of the polymeric material are similar to or approximate those of the tissue into which the implant is to be inserted.

The polymeric material or an implant formed therefrom may further comprise an imaging agent. The imaging agent may conveniently enable the polymeric material or implant to be located in the body via NMR, MRI, X-ray (e.g. CT), ultrasound, infrared (e.g. near-IR), positron emission tomography (PET) imaging, radiography or other imaging techniques. It will be appreciated that a suitable imaging agent can be selected by a skilled person according to the imaging technique desired. For example, the imaging agent may comprise a radiopaque material, a radiotracer, or a fluorescent dye.

In some embodiments the imaging agent comprises a radiopaque material. The imaging agent may be in the form of tags, clips or particles (e.g. a powder). The radiopaque material may be a metal, a metal-containing compound (e.g. a bismuth- or barium-containing compound), an oxide (e.g. MgO), or a bioglass. Suitable metals include titanium (e.g. titanium microparticles), iron, gallium, gadolinium, cobalt, manganese, tungsten, bismuth, barium or the lanthanides.

In some embodiments the imaging agent comprises a radioactive substance, such as a radiotracer or a radiopharmaceutical. Radiotracers typically comprise isotopes with short half-lives, such as carbon-11, nitrogen-13, oxygen-15, fluorine-18, gallium-68, zirconium-89 or rubidium-82. Thus, the radiotracer may be a compound comprising one or more of these isotopes. Other commonly-used radiotracers will be known to those skilled in the art.

In some embodiments the imaging agent comprises a fluorescent dye or probe. Suitable fluorescent dyes include near infra-red fluorophores, such as cyanine dyes (e.g. Cy5 and Cy7).

The imaging agent may be dispersed in the polymeric material e.g. by blended the imaging agent into the resin composition.

Additionally or alternatively, the polymeric material itself may be functionalised such that an implant formed therefrom is detectable in the body using a known imaging technique. In some embodiments, the polymeric material is radiopaque. For example, where an unsaturated side chain is present in the polymeric material (e.g. an unreacted side

chain following cross-linking), the polymeric material may undergo iodination. Thus, in some embodiments the polymeric material comprises iodinated side chains. The presence of iodinated side chains has been found to increase the radiopacity of the polymeric material.

Alternatively or additionally, the polymeric material may be functionalised with one or more metals. For example, the polymeric material may be subjected to post-polymerisation functionalisation in order to attach catechol groups to the crossed-linked polymer which are capable of binding metals. Suitable metals include iron, gallium, gadolinium, cobalt, manganese or the lanthanides.

In some embodiments, the polymeric material or an implant formed therefrom comprises (e.g. is impregnated with, or encapsulates) a biologically active agent, for example a drug or an antimicrobial. The biologically active agent may be dispersed, preferably homogenously, in the polymeric material. For example, a biologically active agent may be added to (*e.g.* mixed into) the resin composition, or the polymeric material formed from the resin composition may be impregnated with a biologically active agent. The biologically active agent may be released from the polymeric material into the surrounding tissue when the implant is *in situ* in the body.

Suitable biologically active agents may include antimicrobials (e.g. antibiotics), anti-inflammatory agents (e.g. a steroid or a non-steroidal anti-inflammatory drug (NSAID)), anti-cancer agents, or growth factors. Growth factors may be selected which are specific for the tissue into which the implant is inserted. Thus, in addition to the void-filling function of the implant, the implant may additionally serve to promote healing and/or reduce inflammation or infection through the release of active agents. The biologically active agents may be small molecules, antibodies, peptides, nucleic acids or proteins. The polymer and/or implant may thus be used for systemic and/or local drug delivery.

In some embodiments, the implant comprises a radioactive material, for example for brachytherapy treatment. The radioactive material may be encapsulated within particles, seeds, ribbons, wires or capsules which are incorporated into the polymeric material, or the implant formed therefrom. Advantageously, this allows radiation to be delivered precisely to the region of tissue surrounding the tumour site, without exposing healthy tissues to radiation. The radioactive material may comprise cesium-131, cesium-137, cobalt-60, iridium-125, iodine-125, palladium-103, ruthenium-106 or radium-226.

Advantageously, the polymeric material or an implant formed therefrom is capable of filling the void left by surgery and promoting faster healing by encouraging the healthy tissue to grow back through the 3-D printed scaffold.

Advantageously, the intricacy of the design of the polymer and/or implant is not limited or constrained by the processability of the resin composition, or the mechanical properties of the resulting polymer.

The polymeric material or implant may be in the form of a mesh, which may also be described as a solid foam. As used herein, the terms "mesh" and "foam" are used interchangeably and refer to a three dimensional network of strands of solid polymeric material which defines and surrounds interconnected gas-filled voids or pores. The interconnectivity of the pores advantageously enables the infiltration of cells and nutrients throughout the implant, thereby facilitating healing and replacement of the mesh with native tissue.

The implant may have a pore size of from 50 μm to 2000 μm, from 100 μm to 1800 μm, from 200 μm to 1500 μm, from 300 μm to 1200 μm, from 400 μm to 1000 μm, from 500 μm to 900 μm, or from 600 μm to 800 μm.

The pore size may vary throughout the foam or mesh or, preferably, all of the pores within the foam or mesh may be substantially the same size.

In some embodiments, the structure of the foam or mesh is uniform in that the strands which form the network surrounding the pores are all of the same thickness. This helps to provide uniform degradation of the implant, in use.

Advantageously, a foam or mesh provides a porous tissue scaffold which promotes healing by promoting cell infiltration and encouraging the healthy tissue to grow back through the pores of the mesh.

The foam or mesh may have any desired geometric structure. For example, the pattern of the network of strands and pores forming the mesh may be based on a crystal lattice structure or a mathematical model. In some embodiments, a suitable mesh may be based on the (10,3)-a network, as described by A. F. Wells (The Third Dimension in Chemistry, 1956). Commercially available software packages can be used to create a description of the desired geometry, which can then be sent to a 3-D printer for fabrication of the mesh.

The implant may have any convenient shape. For example, the implant may have the shape of a sphere, a cube, a cuboid, a pyramid, a cylinder, a cone, a tetrahedron, a prism (e.g. triangular), or any alternate shape.

In some embodiments, the implant comprises an outer coating or skin. The outer coating may cover substantially the whole of the outer surface of the implant. Conveniently, an outer coating may help to avoid point loads being applied to the adjacent tissue. It will be appreciated that the outer coating must be porous so as to enable the infiltration of cells and nutrients. The outer coating may have a pore size which is substantially the same as the pore size of the foam or mesh, or it may have a pore size which is smaller than that of the foam or mesh.

The implant may be of any suitable size for filling a tissue void. For example, in its permanent or expanded state, the implant may have a volume of from 1 to 500 $cm^2$, from 5 to 400 $cm^2$, from 10 to 300 $cm^2$, from 15 to 200 $cm^2$, from 20 to 150 $cm^2$, from 30 to 100 $cm^2$, from 40 to 80 $cm^2$ or from 50 to 70 $cm^2$. .

The pores or voids within the mesh or foam may constitute up to 10%, up to 20%, up to 30%, up to 40%, up to 50%,

up to 60%, up to 70%, up to 75%, up to 80% or up to 90% of the overall volume of the implant.

The polymeric material or an implant formed therefrom is preferably resorbable, e.g. bioresorbable, i.e. the material degrades and is dissolved, excreted or absorbed by the body, as opposed to remaining inert at the implant site. The polymeric material may degrade into non-toxic degradation products that are metabolised or excreted under physiological conditions without causing harm.

A range of degradability time scales may be achieved, wherein the rate of degradation of the polymeric material may be tuned or controlled by controlling the amount or number of carbonate linkages in the composition and/or by modifying the resin composition to result in a different polymer structure. For example, the diluent composition and concentration may be modified to control the rate of degradation of the resulting polymer.

The polymeric material or an implant formed therefrom may have an elastic modulus of from 5 MPa to 4 GPa. The elastic modulus is a measure of an object's or substance's resistance to being deformed elastically (i.e. non-permanently) when a stress is applied to it. For example, the elastic moduli may be from 5 MPa to 3000 MPa, from 8 MPa to 2000 MPa, from 10 MPa to 1000 MPa, from 12 MPa to 850 MPa, from 15 MPa to 500 MPa, from 20 MPa to 400 MPa, from 30 MPa to 300 MPA, from 40 MPa to 200 MPa, from 50 MPa to 150 MPa, or from 70 MPa to 100 MPa.

The polymeric material or an implant formed therefrom may have a compressive modulus of from 0.5 to 50 MPa, from 0.7 to 30 MPa, from 1.0 to 20 MPa, from 1.5 to 18 MPa, from 2.0 to 15 MPa, from 2.5 to 12 MPa, from 3.0 to 10 MPa or from 5.0 to 8 MPa.

The polymeric material or an implant formed therefrom may have a strain to failure value of from 20% to 300%, wherein strain to failure is a measure of how much the implant may be elongated prior to failure. The strain to failure value may be from 30% to 250%, from 40% to 200%, from 50% to 150%, or from 60% to 90%. The inclusion of urethane linkages allows for an increase in strain to failure whilst providing a method of finely tuning the storage and elastic moduli.

The polymeric material and/or implant may exhibit a glass transition temperature (Tg) of between - 10 °C and 150 °C, for example, between 0 to 130 °C, or 5 to 120 °C or 10 to 20 100 °C, or 20 to 80 °C, or 30 to 60 °C, or 35 to 45 °C. For example, the glass transition temperature (Tg) of the cross-linked polymer may be 25, 26, 27, 28, 29, 30, 31, 32, 33, 34, 35, 36, 37, 38, 39, 40, 41, 42, 43, 44, or 45 °C. For example, the glass transition temperature (Tg) of the polymer and/or implant may be between 36.5 and 37.5 °C.

The physical properties of the polymeric material or an implant formed therefrom may be determined using methods known to those skilled in the art, including the methods described herein. As used herein, the term "ambient" refers to a temperature of approximately 22°C.

The polymeric material, or the implant formed therefrom, may have a strain recovery rate at 37 °C of from 10 seconds to 2 hours, from 20 seconds to 90 minutes, from 30 seconds to 60 minutes, from 1 minute to 45 minutes, from 2 minutes to 30 minutes, from 3 minutes to 20 minutes, from 4 minutes to 20 minutes or from 5 minutes to 10 minutes.

The polymeric material, or the implant formed therefrom, may have a peak expansion force of from 0.15 to 1.5, from 0.2 to 1, from 0.25 to 0.9, from 0.3 to 0.8, from 0.4 to 0.7 or 0.8 or from 0.5 to 0.6 or 0.8N at 37 °C.

Expansion force is measured using an implant or scaffold and an alginate gel to mimic mammalian soft tissue. A cubic scaffold (dimensions a x a x a) fixed at 60% strain is inserted into an almond or eye-shaped void or opening (to mimic a surgical void after lumpectomy surgery) with a length of 1.7a and a maximal width of 0.5a. The objective being to have an opening which is of the same volume as the expected volume of the implant or scaffold upon expansion. Once the implant has fully expanded (e.g. upon exposure to a suitable stimulus), or at least at the point of peak expansion, a comparison is made using a thin walled FEA model, wherein a simulated void is subjected to a 1N internal force and the force is then scaled until simulated deformation matches experimental results to provide the expansion force.

It is also possible to determine void filling efficiency using a similar void and implant as set out above (*i.e.* an almond-shaped void or opening of the same volume as the expected volume of the implant) and comparing the actual amount of the void which is filled upon peak expansion of the implant or scaffold. Preferably, the void filling efficiency of the scaffold or implant is greater than 85%, for example, greater than 86, 87, 88, 89, 90, 91, 92, 93, 94, 95, 96, 97, 98, 99, 100%. For example the void filling efficiency may be 85 to 105%, for example 85 to 100%. A void filling efficiency above 100% may be caused by excess expansion *in situ* (*i.e.* when exposed to a stimulus). In practice a void filling efficiency of say 105% (*e.g.* 100-105%) may be tolerable but is preferably avoided.

The polymeric material or an implant formed therefrom may be cyto-compatible. Preferably, the polymeric material or implant is cyto-compatible across multiple cell lines, for example across different human cell lines, and/or across both murine and human cells.

The polymeric material or an implant formed therefrom may have an *in vivo life* of at least 4 weeks, at least 8 weeks, at least 3 months, at least 6 months, at least 8 months or at least 12 months. The *in vivo* half-life may be no more than 36 months, no more than 30 months, or no more than 26 months e.g. 18 to 24 months. An *in vivo* life of 24 months means that the implant has completely degraded and been replaced by a patient's own cells/tissue by 24 months, i.e. has a bio-resorption rate of 24 months.

The polymeric material or an implant formed therefrom may be radiopaque, i.e. the implant may be dense and resist passage of X-rays or similar radiation. The polymeric material and/or implant may therefore provide radiotherapy

guidance post-surgery, enhancing radio-targeting capabilities for surgeons.

The invention thus provides a soft tissue biomaterial which can be formed as an elastic, compliant, degradable void-filling 3D structure that can facilitate infiltration.

Advantageously, the polymer and/or implant of the invention brings a new dimension to 3D printed biodegradable medical devices, with the tuneable biodegradability introducing a temporal/4th dimension of 4D medicine, wherein the tuneable mechanical properties include: mechanical variations including flexibility and strength, a range of degradability time scales, defined bio-resorption rates and/or shape memory with the ability of the material to be compressed for delivery and then expanded to its original shape after exposure to a stimuli.

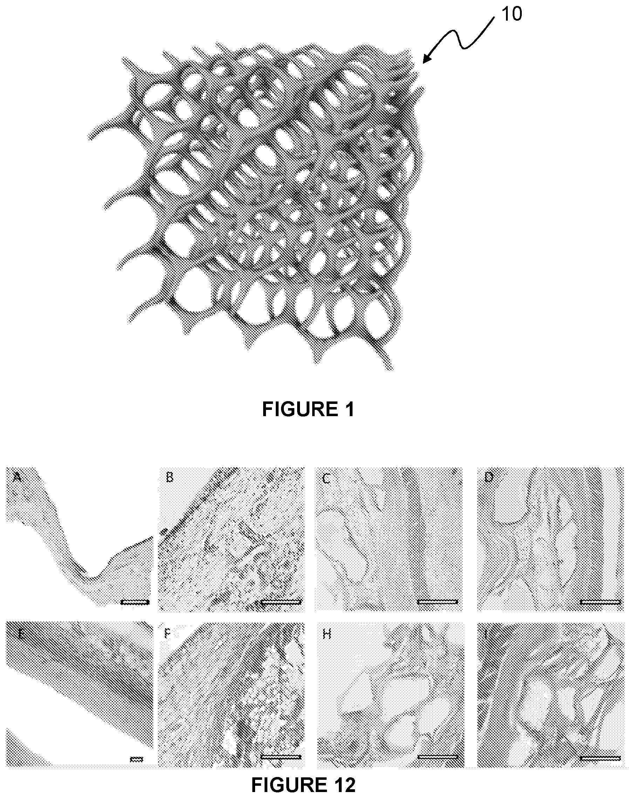

Referring now to Figure 1, there is shown an implant 10 formed from a polymeric material with shape memory properties, according to embodiments of the invention. The implant 10 was printed using a microstereolithographic process. The resin composition was contacted with a photoinitiator, and the microstereolithography apparatus provided the UV light necessary to cure the resin composition into the polymeric material of the implant 10. The implant 10 was printed using a microstereolithographic process. The implant 10 is porous, and may be used as tissue scaffold, for example.

Advantageously, when the resin compositions are printed using microstereolithography, no photoinhibitor is needed to achieve the desired resolution, and print times were averaged at 10 to 30 seconds per slice, with more porous, i.e. smaller struts and lower porosity, materials required longer exposure times.

The implant 10 was printed with a range of pore sizes ranging from 200 μm to 1500 μm. Advantageously, this has been shown to provide an ideal pore size range for a range of biomedical applications, e.g. wherein the implant 10 is a tissue scaffold, for cell growth. Porosities ranging from 0.7 to 0.95 were achievable based on 10,3 tessellation geometry.

Advantageously, using a microstereolithographic process with the resin compositions of the present invention, the design of the implant 10 may be manipulated to provide different surface area, pore interconnectivity, specific morphology. More advantageously, the intricacy of the design of the implant 10 is not limited or constrained by the processability of the resin composition, or the mechanical properties of the resulting polymeric material. Design manipulation of the implant for fabrication using a microstereolithographic process may be achieved using image manipulation and freeware design software. Advantageously, this method of fabricating the polymeric material or an implant formed therefrom is reproducible, using resin compositions, e.g. polymeric materials fabricated from prepolymers and cross-linker pentaerythritol tetrakis(3-mercaptopropionate) in a ratio of 1:1 ene to thiol, the prepolymers fabricated from first cyclic carbonate comprising the formula (x)

(x)

wherein the only variable was the exposure time of the UV light to the resin composition to cure the cross-linked polymer.

## Kit

According to an aspect of the invention there is provided a kit for reconstruction of tissue (e.g. soft tissue) following a surgical procedure, the kit comprising at least one implant, and instructions for use. The implant is one as described herein.

In some embodiments, the kit is for the reconstruction of soft tissue, for example a breast following a lumpectomy procedure. In some embodiments the kit is for repairing hard tissue, such as bone, for example following trauma or surgery.

More than one implant may be required following a surgical procedure. The kit may comprise at least two implants, e.g. three, four or five implants.

Where more than one implant is provided the implant or each implant may differ from each other in at least their size, shape, material or mechanical properties, e.g. elastic moduli, storage moduli, strain to failure, density and/or porosity.

In some embodiments, the kit comprises a first implant, a second implant and a third implant, wherein the second implant is greater in volume than the first implant, and the third implant is greater in volume the second implant.

The kit may further comprise an instrument for insertion of the implant.

In some embodiments, the kit further comprises apparatus for compressing the implant. The implant may be provided in an expanded state and may therefore require compression prior to insertion into the void.

In some embodiments, the kit further comprises stimulating device or reagent, for applying an external stimulus to the implant so as to cause it to change state, for example from a temporary (e.g. compressed) state to a permanent (e.g. expanded) state. For example, a stimulating device may comprise a heater, such as a laser. A stimulating reagent may be water. It will be appreciated that the type of stimulus required to cause the implant to change state, and thus the nature of the stimulating device or reagent, will be selected by the skilled person according to the chemical properties of the polymeric material from which the implant is formed.

## Method of manufacture

In a further (unclaimed) aspect, the invention provides a method of manufacturing an implant (e.g. a void occlusion device), the method comprising:

(i) providing a resin composition comprising a prepolymer and optionally one or more diluent(s) ;
(ii) shaping the resin composition into the desired shape of the implant; and
(iii) cross-linking the prepolymer, thereby forming the implant.

It will be appreciated that the implant formed in step (iii) comprises a cross-linked polymeric material.

In some embodiments steps (ii) and (iii) may be carried out simultaneously.

The polymeric material or an implant formed therefrom may be fabricated using an additive manufacturing technique or apparatus. In some embodiments, the step (ii) of shaping the resin composition is carried out by 3D printing. In some embodiments, both steps (ii) and (iii) are carried out by 3D printing.

In some embodiments the implant is formed by 3D bioplotting or inkjet printing.

The implant may be fabricated using stereolithography or microstereolithography. For example. In some embodiments, the steps (ii) of shaping the resin composition and (iii) cross-linking the prepolymer are carried out using stereolithography or microstereolithography. For example, the resin composition may comprise or be contacted with a photoinitiator, and the method may comprise using a microstereolithography apparatus to provide the UV light necessary to cure the resin composition into the polymeric material. In some embodiments, steps (ii) and (iii) are carried out using digital light processing (DLP).

Advantageously, using a microstereolithographic process with the resin compositions of the present invention allows for rapid iteration of the product design, consistent accurate production and the ability to customise production to meet the needs of individual clients, i.e. the design of the implant may be manipulated to provide different surface area, pore interconnectivity and/or specific morphology.

In some embodiments, the resin composition has a viscosity of no more than 20 Pa.s, no more than 15 Pa.s no more than 12 Pa.s. or no more than 10 Pa.s at 22 °C. The viscosity of the resin composition can be determined using methods known to the skilled person, for example rheology as described herein.

In some embodiments, the method further comprises modifying the implant, for example to further optimise the shape or size of the implant. The implant may be modified using machining techniques, for example, turning, milling, sanding, filing, cutting and/or drilling.

In some embodiments, the method further comprises joining the implant to one or more other components, or assembling the implant into a complex.

The implant may be a 4D printed device, i.e. the implant may be fabricated using an additive manufacturing technique such as 3D printing to produce a primary shape (e.g. an expanded form), which may be further deformed to produce a secondary shape (e.g. a compressed form). The secondary shape may a compact, flexible and/or deployable shape, for example, a minimally invasive shape for minimally invasive delivery to a site within a patient.

Therefore, in some embodiments the method further comprises deforming the implant, e.g. compressing the implant. The implant may be compressed by applying a force to the implant. For example, the implant may be compressed by hand, or by compressing between two opposing plates.

The method may further comprise adding a biologically active agent and/or an imaging agent to the resin composition and/or to the polymeric material or implant. The imaging agent may be one as described herein above. In some embodiments, the method comprises blending the resin with an imaging agent, for example titanium microparticles or magnesium oxide.

In some embodiments, the method further comprises adding a radioactive material to the polymeric material or implant. The radioactive material may be encapsulated within particles, seeds, ribbons, wires or capsules which are incorporated into the polymeric material or implant.

The method may further comprise preparing the resin composition. The resin composition may be prepared by mixing a prepolymer with one or more reactive diluents. The resin composition may further comprise one or more chain

extenders. The resin composition may further comprise one or more photoinitiators and, optionally, one or more photoinhibitors.

In a preferred embodiment, the resin composition comprises:

g. a prepolymer, optionally poly(TMPAC), poly(NTC) or poly(TMPAC-co-NTC);
h. a reactive diluent, optionally a diluent containing urethane;
i. a cross-linking agent, e.g. PETMP;
j. a photoinitiator, optionally one that is active at a wavelength of from 100 to 700 nm, from 120 to 650 nm, from 150 to 600 nm, from 180 to 500 nm, from 200 to 450, from 250 to 400 or from 300 to 350, for example 350 to 450 nm (e.g. 405 nm);
k. a photoinhibitor, optionally one with competitive absorbance in substantially the same region as the photoinitiator.

In some embodiments, the method further comprises functionalising the cross-linked polymeric material of the implant, after cross-linking (i.e. after step (iii)).

## Resin composition

The resin composition comprises a prepolymer and optionally one or more diluent(s), the prepolymer comprising repeating units having at least one carbonate linkage and at least one unsaturated side chain, the at least one optional diluent(s) comprising at least one unsaturated side-chain, wherein either or both of the prepolymer and the at least one optional diluent(s) comprises at least one O=C-N linkage. The O=C-N linkage may be one of a urethane linkage, and/or a urea linkage, preferably a urethane linkage.

In some embodiments, the resin composition comprises more than one diluent, for example two diluents, three diluents, four diluents, or more than four diluents. Each diluent may comprise at least one unsaturated side-chain, preferably plural unsaturated side chains.

In embodiments, the resin composition may comprise a prepolymer containing carbonate and urethane linkages and unsaturated side chains which are capable of being crosslinked; at least one cross linker capable of reacting with at least two unsaturated side chains of the prepolymer and, optionally, a cross linkable diluent or diluents comprising at least 2 unsaturated side chains.

In embodiments, the resin composition may comprise a prepolymer having repeating units,

the repeating units comprising at least one carbonate linkage, at least one urethane linkage,
and at least one unsaturated side-chain. In embodiments, the resin composition may further comprise a cross-linker.

In embodiments, the or each diluent may comprise a urethane linkage and/or a urea linkage. Preferably, the or each diluent comprises a urethane linkage.

In embodiments, the unsaturated side-chain of the prepolymer and/or the diluent comprises an aliphatic moiety (e.g. an alkene, an alkyne), or an aromatic moiety, for example, a phenyl group or a substituted phenyl group, a heterocyclic aromatic moiety, or a polycyclic aromatic hydrocarbon. The unsaturated side-chain may be linear or may be cyclic.

Alternatively, one, some or all of the diluents may comprise plural moieties, for example a side chain comprising one or more different moieties, i.e. a moiety other than an unsaturated side-chain.

The cross-linker may comprise a moiety that is capable of reacting with an unsaturated side-chain of the prepolymer and/or the diluent. For example, the cross-linker may comprise an azide moiety that is capable of reacting with an alkyne moiety on a side chain of the one or more diluents and/or the prepolymer. The cross-linker comprises a thiol group that is capable of reacting with an alkene moiety on a side chain of the one or more diluents and/or the prepolymer.

Alternatively, one, some or all of the diluents may comprise a side chain comprising a moiety other than an unsaturated side-chain, the moiety being capable of reacting with a moiety on the cross-linker to produce a covalent bond between the cross-linker and the diluent. For example, the cross-linker may comprise an unsaturated side-chain (e.g. an alkyne or alkene), and the or each of the diluents may comprise a side chain having an azide group. Alternatively, the cross-linker may comprise an alkene moiety and the or each diluent may comprise a side chain having a thiol moiety.

The, or some or all of the diluents may comprise one or more allyl groups. For example, the diluent may comprise two allyl groups, or three allyl groups, or four allyl groups. The diluent may comprise the general formula (i):

(i)

Y

wherein Y comprises an alkyl and/or an aryl moiety, or a functionalised alkyl and/or a functionalised aryl moiety. For example, Y may comprise an alkyl chain comprising 1 to 15 carbons, for example 1 to 10 carbons, or 1 to 5 carbons. For example, Y may comprise an alkyl chain comprising 1, 2, 3, 4, 5, 6, 7, 8, 9, or 10 carbons.

In embodiments, one, some or all of the diluents may comprise two or more unsaturated side-chains. The unsaturated side chains may comprise an alkene moiety. For example, a diluent may be selected from one or more of the following: (ii) 1,3,5-triallyl-1,3,5-triazine2,4,6(1H,3H,5H)-trione, (iii) 6-(allyloxycarbonylamino)hexylamino 3- butenoate, (iv) 3-[(allyloxycarbonylamino)methyl]-3,5,5-trimethylcyclohexylamino 3-butenoate, and (v) diallyl phthalate:

(ii) (iii)

(iv) (v)

In embodiments, the diluent may be propylene carbonate.

The cross-linker comprises one or more thiol moieties, for example, one thiol moiety, two thiol moieties, three thiol moieties, or four moieties, or more than four moieties. In embodiments, the cross-linker has a molecular weight of between 100 to 800 g/mol, for example, between 200 to 700 g/mol, or 300 to 600 g/mol, or 400 to 500 g/mol.

The cross-linker may be pentaerythritol tetrakis(3-mercaptopropionate) (PETMP), comprising the formula (vi):

(vi)

The thiol moieties of the cross-linker (vi) are capable of reacting with unsaturated moieites, specifically unsaturated side-chains of the prepolymer (vii, shown below) and of the diluents (1,3,5-triallyl-1,3,5-triazine2,4,6(1H,3H,5H)-trione, 6-(allyloxycarbonylamino)hexylamino 3- butenoate, 3-[(allyloxycarbonylamino)methyl]-3,5,5-trimethylcyclohexylamino 3-butenoate, and diallyl phthalate.

In embodiments, the prepolymer may comprise the formula (vii):

(vii)

wherein R group is an aliphatic or an aromatic moiety or group, $R^1$ is an aliphatic or an aromatic moiety or group, $R^2$ is an aliphatic or an aromatic moiety or group, $R^3$ is an aliphatic or an aromatic moiety or group, and $R^4$ is an aliphatic or an aromatic moiety or group, and wherein x is a number that is one or greater and less than one hundred, e.g. 99, 98, 97, 96, 95, 94, 93, 92, 91, 90, 80, 70, 60, 50, 40, 30, 20, or 10.

In embodiments, the prepolymer may comprise the formula (viii):

(viii)

wherein the R group is an aliphatic or an aromatic moiety or group, and wherein x is a number that is one or greater and less than one hundred, e.g. 99, 98, 97, 96, 95, 94, 93, 92, 91, 90, 80, 70, 60, 50, 40, 30, 20, or 10.

In an embodiment, R is an alkyl group comprising six carbons.

In embodiments, the prepolymer may be a copolymer.

In embodiments, the prepolymer may be fabricated from components comprising the formulae (ix) and a diisocyanate (I):

(ix)

(I) O=C=N-R-N=C=O

wherein R group is an aliphatic or an aromatic moiety or group, $R^1$ is an aliphatic or an aromatic moiety or group, $R^2$ is an aliphatic or an aromatic moiety or group, $R^3$ is an aliphatic or an aromatic moiety or group, and $R^4$ is an aliphatic or an aromatic moiety or group, and wherein x is a number that is less than one hundred, e.g. 99, 98, 97, 96, 95, 94, 93, 92, 91, 90, 80, 70, 60, 50, 40, 30, 20, or 10.

In embodiments, any or all of $R^1$, $R^2$ , $R^3$ and/or $R^4$ may be a hydrogen atom, an alkyl chain, e.g. methyl, ethyl, propyl, butyl and so on, and isomers thereof; an aromatic ring, an aliphatic ring, an allyl ether, an acrylate (e.g. with modification), and/or an allyl ester.

In embodiments wherein R, $R^1$, $R^2$, $R^3$, and/or $R^4$ is an aromatic group, the aromatic group may be one of, or a combination of, an aromatic hydrocarbon group, and/or an aromatic heterocyclic group.

In embodiments wherein R, $R^1$, $R^2$, $R^3$, and/or $R^4$ is or comprises an aromatic hydrocarbon group, the aromatic hydrocarbon group may comprise one of, or a combination of, a phenyl ring and/or a substituted phenyl ring. There may be one, two, three, four, or five additional substituents on the phenyl ring. The substituents are bonded directly to the phenyl ring, and may be one of, or a combination of, fluorine, chlorine, bromine, iodine, a hydroxyl group, an amine group, a nitro group, an alkoxy group, a carboxylic acid, an amide, a cyano group, a trifluoromethyl, an ester, an alkene an alkyne, an azide, an azo, an isocyanate, a ketone, an aldehyde, an alkyl group consisting of a hydrocarbon chain, or a hydrocarbon ring, an alkyl group consisting of other heteroatoms such as fluorine, chlorine, bromine, iodine, oxygen, nitrogen, and/or sulphur. The alkyl group may comprise a hydroxyl group, an amine group, a nitro group, an ether group, a carboxylic acid, an amide, a cyano group, trifluoromethyl, an ester, an alkene an alkyne, an azide, an azo, an isocyanate, a ketone, an aldehyde, for example. The substituents may be another aromatic group, for example, R, $R^1$, $R^2$, $R^3$, and/or $R^4$ may comprise a phenyl substituted with a further phenyl ring. In embodiments, the R, $R^1$, $R^2$, $R^3$, and/or $R^4$ group may be a phenyl ring, substituted with a second phenyl ring, which in turn is substituted with a third phenyl ring.

In embodiments wherein R, $R^1$, $R^2$, $R^3$, and/or $R^4$ is an aromatic group, the aromatic group may be a polycyclic aromatic hydrocarbon, for example, naphthalene, anthracene, phenanthrene, tetracene, chrysene, triphenylene, pyrene, pentacene, benzo[a]pyrene, corannulene, benzo[ghi]perylene, coronene, ovalene, fullerene, and/or benzo[c]fluorene. The R group may be bonded to the triphenylene derivative by any isomer of the polycyclic aromatic hydrocarbons described, for example, 1-napthalene, 2-napthalene, 2-anthracene, 9-anthracene. The polycyclic aromatic hydrocarbon group may be substituted with other moieties such as aryl groups, alkyl groups, heteroatoms, and/or other electron withdrawing or electron donating groups.

In embodiments wherein R, $R^1$, $R^2$, $R^3$, and/or $R^4$ is an aromatic heterocyclic group, the heterocyclic group may be a four membered ring, a five membered ring, a six membered ring, a seven membered ring, an eight membered ring, a nine membered ring, a ten membered ring, or a fused ring. In embodiments, the heterocyclic group may be furan, benzofuran, isobenzofuran, pyrrole, indole, isoindole, thiophene, benzothiophene, benzo[c]thiophene, imidazole, benzimidazole, purine, pyrazole, indazole, oxazole, benzoxazole, isoxazole, benzisoxazole, thiazole, benzothiazole, pyridine, quinoline, isoquinoline, pyrazine, quinoxaline, acridine, pyrimidine, quinozoline, pyridazine, cinnoline, phthalazine, 1,2,3-triazine, 1,2,4-triazine, 1,3,5-triazine.pyridine or thiophene.

In embodiments wherein R, $R^1$, $R^2$, $R^3$, and/or $R^4$ is an aliphatic group, the aliphatic group may be one of, or a combination of, an n-alkyl chain, a branched alkyl chain, an alkyl chain comprising unsaturated moieties, an alkyl chain comprising heteroatoms, for example, fluorine, chlorine, bromine, iodine, oxygen, sulphur, nitrogen. The alkyl chain may comprise unsaturated portions, comprising alkenes, or aromatic moieties. The alkyl chain may comprise functional groups for further derivatisation of the iphenylene derivative. For example, the functional groups may be one or more of an azide, a carbonyl group, an alcohol, a halogen, or an alkene.

R, $R^1$, $R^2$, $R^3$, and/or $R^4$ may comprise an aliphatic ring, or an aromatic ring. R, $R^1$, $R^2$, $R^3$, and/or $R^4$ may comprise an allyl ether, an acrylate, a modified acrylate, and/or an allyl ester. R, $R^1$, $R^2$, $R^3$, and/or $R^4$ may comprise a spirocyclic aliphatic ring, and/or a bridged ring, e.g.a norbornene ring.

We prefer R to be an aliphatic moiety.

In embodiments, the prepolymer has a molecular weight of up to 3 kDa, for example up to 1 kDa, or 2 kDa. The prepolymer may comprise a polydispersity index (PDI) of approximately 1.4.

In embodiments, the prepolymer may be a polycarbonate. In embodiments, the prepolymer may not comprise a urethane linkage and/or any other O=C-N linkage. The prepolymer may be a homopolymer of 5-[(allyloxy)methyl]-5-ethyl-1,3-dioxan-2-one. Additionally or alternatively, the prepolymer may be a homopolymer of 9-(5-norbornen-2-yl)-2,4,8,10- tetraoxa-3-spiro[5.5]undecanone. The prepolymer may comprise a copolymer of 5-[(allyloxy)methyl]-5-ethyl-1,3-dioxan-2-one and 9-(5-norbornen-2-yl)-2,4,8,10-tetraoxa-3-spiro[5.5]undecanone.

In embodiments, the prepolymer may be chain extended using an isocyanate compound to create a urethane linkage. The isocyanate compound preferably comprises two or more isocyanate moieties. For example, the isocyanate may be isophorone diisocyanate (IPDI). In alternative embodiments, the isocyanate is hexamethylene diisocyanate (HDI). However, any suitable diisocyanate may be used, e.g. tetramethylxylene diisocyanate (TMXDI), phenylene diisocyanate, toluene diisocyante (TDI), xylylene diisocyanate (XDI), cyclohexylene diisocyanate and so on.

The resin composition may comprise the prepolymer being present in a quantity of between 10 and 100 w/w% of the total composition, for example, between 20 and 90 w/w%, or 40 and 80 w/w%, or 60 and 70 w/w%. For example, the resin composition may comprise the prepolymer in a quantity of 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, or 75 w/w%. In embodiments, the resin composition comprises the prepolymer is present in the resin composition in a quantity of 60 w/w%.

In embodiments, the total quantity of diluent may be present in a quantity of between 0 and 50 w/w% of the total composition, for example, between 5 and 45 w/w%, or 10 and 40 w/w%, or 15 and 35 w/w%, or 20 and 30 w/w% or 25 w/w%. For example, the resin composition may comprise a total quantity of diluent of 20, 21, 22, 23, 24, 25, 26, 27, 28, 29, 30, 31, 32, 33, 34, 35, 36, 37, 38, 39, or 40 w/w%.

The cross-linker may be present in a quantity of between 0 and 50 w/w% of the total composition, for example, between 5 and 45 w/w%, or 10 and 40 w/w%, or 15 and 35 w/w%, or 20 and 30 w/w% or 25 w/w%. For example, the resin composition may comprise a total quantity of cross-linker of 20, 21, 22, 23, 24, 25, 26, 27, 28, 29, 30, 31, 32, 33, 34, 35, 36, 37, 38, 39, or 40 w/w%

The method may further comprise fabricating a prepolymer.

In some embodiments the prepolymer (C) is fabricated according to the following method:

i. providing an oligomer of formula (A);
ii. providing a reagent of the formula (B), wherein the reagent (B) comprises two or more isocyanate moieties;
iii. reacting the oligomer (A) with the reagent (B) to fabricate the prepolymer (C),

wherein R group is an aliphatic or an aromatic moiety or group, $R^1$ is an aliphatic or an aromatic moiety or group, $R^2$ is an aliphatic or an aromatic moiety or group, $R^3$ is an aliphatic or an aromatic moiety or group, and $R^4$ is an aliphatic or an aromatic moiety or group, and wherein x is a number that is one or greater and less than one hundred, e.g. 99, 98, 97, 96, 95, 94, 93, 92, 91,90, 80, 70, 60, 50, 40, 30, 20, or 10.

The prepolymer may be fabricated in a chain extension reaction from polycarbonate oligomer (A) and diisocyanate (B).

The prepolymer (C) may be a mixed polycarbonate polyurethane oligomer.

The diisocyanate (B) may be isophorone diisocyanate (IPID).

In an embodiment the polycarbonate (A) is synthesised in a ring opening polymerisation reaction from a first cyclic carbonate and a second cyclic carbonate, in the presence of water and a DBU initiator. The reaction of the first cyclic carbonate and the second cyclic carbonate yielding oligomers of polycarbonate (A) with lengths of below 1.2 kDa with PDIs of below 1.2.

Advantageously, organocatalytic ring opening polymerization (ROP) of aliphatic cyclic carbonates achieves degradable polymer backbones without acidic degradation, while maintaining good control over the synthesis.

In an alternative embodiment, the prepolymer (not shown) may be fabricated from the first cyclic carbonate only. In an alternative embodiment, the prepolymer (not shown) may be fabricated from the first cyclic carbonate only. These may or may not be chain extended using a diisocyanate.

In some embodiments there is provided a method of forming a polymer, the polymer comprising at least one unsaturated side-chain, the method comprising:

i. providing a resin composition, the resin composition comprising a prepolymer and optionally one or more diluent(s), the prepolymer comprising repeating units having at least one carbonate linkage and at least one unsaturated side-chain, the at least one optional diluent(s) comprising at least one unsaturated side-chain, wherein either or both of the prepolymer and the at least one optional diluent(s) comprises at least one O=C-N linkage, preferably a urethane linkage;
ii. shaping the resin composition into the desired shape of the implant; and
iii. cross-linking the prepolymer.

In some embodiments, prepolymer A is combined with cross-linker (vi) and one or more of the diluents, 1,3,5-triallyl-1,3,5-triazine2,4,6(1H,3H,5H)-trione, 6-(allyloxycarbonylamino)hexylamino 3-butenoate, 3-[(allyloxycarbonylamino)methyl]-3,5,5- trimethylcyclohexylamino 3-butenoate, and diallyl phthalate, to produce a range of resin compositions, for fabrication into crosslinked polymers according to the invention.

The components of the resin compositions, i.e. the prepolymer, the diluents, and/or the cross-linker, for fabricating the cross-linked polymers of the invention may be added in different amounts to tune or vary the properties, e.g. degradability, shape memory properties, of the resulting cross-linked polymer. In embodiments wherein the prepolymer comprises a urethane linkage, the quantity of the diluent in the resin composition may be 0 wt.%. In this case, the prepolymer may be capable of directly cross-linking to moieties on or within the prepolymer itself and/or to a cross-linker.

Advantageously, the type of prepolymer and/or reactive diluent and/or cross-linker that is added to the resin composition to fabricate the cross-linked polymers of the invention may be varied to tune the properties of the cross-linked polymer. For example, the structure of the prepolymer may be varied by using different types and/or concentrations of monomer to fabricate the prepolymer. In embodiments, the prepolymer is fabricated from one type of carbonate monomer.

In other embodiments, the prepolymer is fabricated from more than one type of carbonate monomer. The concentration of each monomer in the prepolymer may be adjusted or varied to tune the properties of the resulting cross-linked polymer. In embodiments, the prepolymer may be chain extended using an isocyanate to provide a urethane linkage in the prepolymer. The type of isocyanate in the prepolymer may be varied to tune the properties of the resulting cross-linked polymer that is fabricated from a resin composition containing the prepolymer.

In an embodiment, the cross-linked polymer of the invention comprises one or more urethane and/or urea linkage. The origin of the urethane linkage is from one or more of a urethane linkage in the prepolymer and/or one or more diluents 6-(allyloxycarbonylamino)hexylamino 3- butanoate and/or 3-[(allyloxycarbonylamino)methyl]-3,5,5- trimethylcyclohexylamino 3-butenoate. For example, the prepolymer need not comprise a urethane linkage, e.g. the prepolymer may be a polycarbonate that consists of carbonate linkages only. In this case, the origin of the urethane and/or urea linkage(s) is from the diluents 6-(allyloxycarbonylamino)hexylamino 3- butanoate and 3-[(allyloxycarbonylamino)methyl]-3,5,5- trimethylcyclohexylamino 3-butenoate only.

In an alternative embodiment, the prepolymer for use in the resin compositions of the invention may comprise carbonate linkages in addition to one or more urethane linkages. In this case, the origin of the urethane and/or urea linkage(s) is from the prepolymer (e.g. prepolymer C) and may also be (but need not be) from the diluents 6-(allyloxycarbonylamino)hexylamino 3- butanoate and/or 3-[(allyloxycarbonylamino)methyl]-3,5,5- trimethylcyclohexylamino 3-butenoate.

In an embodiment, the mechanism of step (iii) is a radical alkene mechanism, a radical alkyne mechanism, a nucleophilic alkene mechanism or a nucleophilic alkyne mechanism.

The cross linker may comprise multiple thiol moieties, said thiol moieties of the cross-linker may react with the unsaturated side-chains of the prepolymer and/or the diluent(s), wherein the unsaturated side-chains comprise an alkene moiety, and the resin composition is combined with a radical initiator, e.g. a photoinitiator, then the cross-linking reaction between oligomer chains of the prepolymer and the cross-linker and/or the diluents, may proceed via an radical alkene mechanism.

Wherein the unsaturated side-chains comprise an alkyne moiety, and the resin composition is combined with a radical initiator, e.g. a photoinitiator, then the cross-linking reaction between oligomer chains of the prepolymer and the cross-linker and/or the diluent(s) may proceed via a radical alkene mechanism.

In contrast, the unsaturated side-chains of a prepolymer and/or a or the diluent(s) may comprise an alkene moiety comprising an electron withdrawing group, which may undergo a nucleophilic addition reaction with the cross-linker, in a nucleophilic alkene mechanism.

Alternatively, unsaturated side-chains of a prepolymer and/or a or the diluent(s) may comprise an alkyne moiety comprising an electron withdrawing group, which may undergo a nucleophilic addition reaction with the cross-linker, in a nucleophilic alkyne mechanism.

The cross-linking processes described above may be performed on an apparatus for microstereolithography (not shown), which 3D prints each layer of the cross-linked polymer, by providing an initiator, e.g. a photoinitiator and a light source, to cure the cross-linked polymer.

Advantageously, the quantity of prepolymer and/or diluent and/or cross-linker may be altered to afford a range of cross-linked polymer with different properties, e.g. mechanical properties, glass transition temperatures (Tg), degradability, and so on. In this way, the properties of the cross-linked polymer of the present invention may be tuned depending on the application. The type of diluent(s) may also be varied to afford crosslinked polymers with different properties.

Preferably, step (iii) cross-linking the pre-polymer is performed by contacting the resin composition with an initiator. Preferably, an energy source is provided to activate the initiator.

The method may comprise contacting the resin composition with a catalyst and/or an initiator. For example, the catalyst and/or initiator may be a photoinitiator. The method may comprise exposing the resin composition comprising a photoinitiator to an energy source, for example, a light source, for example, UV light. For example, polymerisation of the carbonate monomer may be achieved in an organocatalyzed reaction using a DBU (1,8-diazabicyclo[5.4.0]undec-7-ene) initiator in water.

The initiator may be a photoinitiator, e.g. a bis acyl phosphine. Suitable photoinitiators include those sold under the trade name Irgacure (RTM) by BASF, for example, Irgacure 819, or those sold under the trade name Omnicat (RTM) photoinitiators by IGM resins.

The initiator may be a radical initiator, for example, a peroxide such as hydrogen peroxide, or an organic peroxide such as benzoyl peroxide. The radical initiator may be an azo compound, for example, AIBN or ABCN. In embodiments, the energy source may be heat, i.e. the reaction may be initiated thermally.

The initiator may be present in a quantity of between 0 and 5 w/w% of the total composition, for example, up to 4 w/w%, or up to 3 w/w%, or up to 2 w/w%, or up to 1 w/w% of the total composition, for example, 0.5 w/w% of the total composition. The initiator, e.g. the photoinitiator, may be present in a quantity of 0.5, 1.0, 1.5, 2.0, 2.5, 3.0, 3.5, 4.0, 4.5, or 5.0 w/w% of the total composition.

The method may be performed in or by an apparatus for 3D printing, e.g. an apparatus for stereolithography.

The cross-linked polymer may be further functionalised. The further functionalisation may take place post polymerisation, i.e. after the cross-linked polymer has been fabricated from the resin composition. The cross-linked polymer may comprise unsaturated side-chains after the cross-linking process has taken place. The method may comprise further functionalisation of these unsaturated side chains. For example, the method may comprise cross-linking a polymer in an additive manufacturing process, e.g. a 3D printing process and/or a stereolithography process, and further providing reagents to functionalise the cross-linked polymer, e.g. the surface of the cross-linked polymer. The functionalisation of the cross-linked polymer may take place in a separate step.

In embodiments, the method may further comprise step iv. providing a reagent for halogenation of at least one unsaturated side chain of the cross-linked polymer. The reagent may be a diatomic halogen, e.g. chlorine, bromine and/or iodine, or a halogenating reagent, e.g. a hypohalous acid such as HOCl, HOBr, HOI, or a Brønsted acid, e.g. HF, HCl, HBr, and/or HI.

Additionally or alternatively, the method may further comprise step v. providing a reagent for alkylation of the at least one unsaturated side chain. The reagent may be an alkylating agent, e.g. an alkyl halide, or an alkyl thiol.

Additionally or alternatively, the method may further comprise step vi. providing a reagent for functionalising the at least one unsaturated side chain with a hydrophobic moiety. The hydrophobic moiety may increase the hydrophobicity of the cross-linked polymer. The hydrophobic moiety may comprise an alkyl chain, for example, a linear alkyl chain comprising between 8 and 15 carbons, say 10 carbons, or 9, 10, 11, 12, 13, 14, or 15 carbons. In embodiments, the reagent may be a compound comprising a thiol moiety, e.g. an alkyl or aryl thiol compound, that is capable of adding across an unsaturated side-chain, e.g. an alkene moiety.

Additionally or alternatively, the method may further comprise a step for providing a reagent for functionalising the at least one unsaturated side chain with a hydrophilic moiety. The hydrophilic moiety may increase the hydrophilicity of the cross-linked polymer. The hydrophilic moiety may comprise one or more carboxylic acid groups, and/or one or more hydroxyl groups. The hydrophilic moiety may comprise an alkyl chain comprising one or more carboxylic acid groups and/or one or more hydroxyl groups. In embodiments, the reagent may be a compound comprising a thiol moiety comprising hydrophilic groups, e.g. an alkyl or aryl thiol compound comprising hydrophilic side groups, that is capable of adding across, and/or reacting with, an unsaturated side-chain, e.g. an alkene moiety to form a covalent bond.

Alternatively, the unsaturated side-chains of the cross-linked polymer may be further functionalised in other types of reaction. For example, the one or more unsaturated side-chain of the cross-linked polymer may be an alkene, and may react in a cycloaddition, e.g. a Diels-Alder reaction. Other atoms or moieties may be added across or to the unsaturated side chains. For example, the unsaturated side-chain may be an alkene that undergoes an epoxidation or a cyclopropanation.

Additionally or alternatively, the method may further comprise a step for providing a reagent for functionalising the at least one unsaturated side chain with a tag, for example, a fluorescent tag, a radioactive tag, or a biomolecule tag, for labelling or detection of the cross-linked polymer. This is particularly useful if the cross-linked polymer is fabricated into a medical device for implantation into a patient.

Additionally or alternatively, the method may further comprise step vii. providing a reagent for functionalising the at least one unsaturated side chain with a biomolecule, for example, a protein, and/or a cell adhesion moiety, e.g. a cell adhesion molecule (CAM). The biomolecule may be involved in adhesion or binding to physiological targets. For example, a cell adhesion molecule (CAM) may be involved in binding to cells, e.g. bone cells within a tissue scaffold, or to the extracellular matrix. For example, the further functionalised crosslinked polymer may comprise a functionalised surface to elicit a specific cellular response.

The steps iv, v, vi, and/or vii of the method may be performed at the same time as the resin composition is fabricated into a cross-linked polymer, e.g. during additive manufacture, or may be performed after the resin composition has been fabricated into a cross-linked polymer in a separate step, i.e. after steps i to iii of the method. Only one of the steps iv, v, vi, and/or vii may be performed after steps i to iii have been performed. Alternatively, two or more of the steps may be selected to be performed, either consecutively or concurrently, after steps i to iii have been performed. For example, the method may comprise steps i to iii, followed by step iv and further followed by step vii.

Additionally or alternatively, the monomers of the prepolymer may undergo further functionalisation. The monomers of the prepolymer may be functionalised before polymerisation into the prepolymer. The monomers of the prepolymer may be functionalised after polymerisation into the prepolymer, but before cross-linking into a cross-linked polymer.

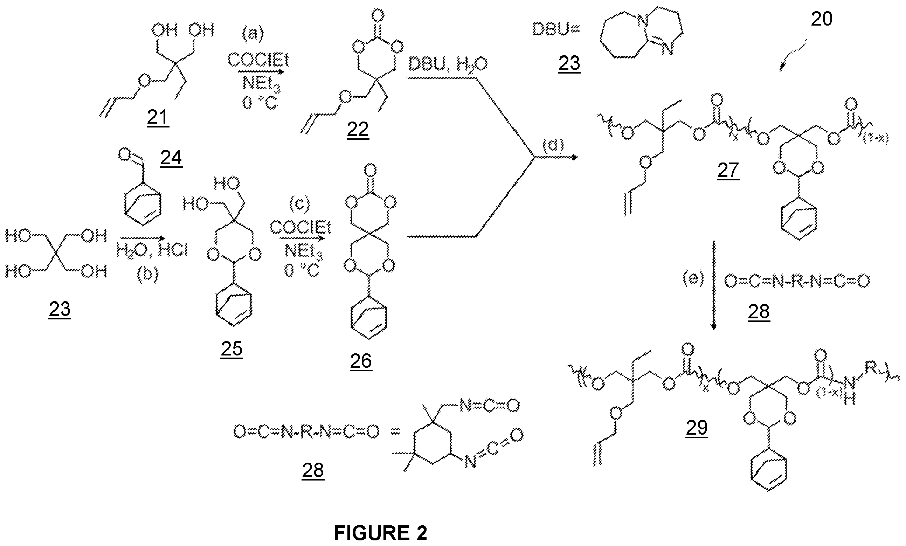

Referring now to Figure 2, there is shown a synthetic route 20 to a prepolymer 29 for use in a resin composition, according to an embodiment of the invention. In an embodiment, the prepolymer 29 can be fabricated in a chain extension reaction (e) from a polycarbonate oligomer 27 and a diisocyanate 28 to produce a mixed polycarbonate polyurethane prepolymer 29. In an embodiment, the diisocyanate 28 may be isophorone diisocyanate (IPDI) 28. The prepolymer 29 may have molecular weights of less than or equal to 3 kDa and polydispersity indices (PDI) of 1.4.

The polycarbonate 27 may be synthesised in a ring opening polymerisation reaction (d) from a first cyclic carbonate 22 and a second cyclic carbonate 26 in the presence of water and a DBU initiator 23. The reaction (d) of first

cyclic carbonate 22 and second cyclic carbonate 26 may yield oligomers of polycarbonate 27 with lengths of below 1.2 kDa with PDIs of below 1.2.

In an embodiment, the first cyclic carbonate 22 may be TMPAC, and the second cyclic carbonate 26 may be NTC. The first and/or second cyclic carbonates may be synthesised in accordance with the protocols described in IA Barker et. al., Biomaterials Science, 2014, 2, 472-475; and also in Y He et. al., Reactive and Functional Polymers, Vol. 71, Issue 2, February 2011, p. 175-186.

First cyclic carbonate 22 can be synthesised in one step, in reaction (a) from diol 21 and propionyl chloride in the presence of triethylamine at 0 °C. In an embodiment, diol 21 is 2-[(allyloxy)methyl]-2-ethyl-1,3-propanediol. Second cyclic carbonate 26 may be synthesised in two steps, using polyol 23 as the starting material. In reaction (b), polyol 23 and aldehyde 24 may undergo reaction in the presence of hydrochloric acid to produce diol 25. Diol 25 may undergo subsequent reaction, in reaction (c), with propionyl chloride in the presence of triethylamine at 0 °C to produce second carbonate 26. In an embodiment, polyol 23 may be pentaerythritol, aldehyde 24 may be bicyclo[2.2.1]hept-5-ene-2-carboxaldehyde, and diol 25 may be [5-(hydroxymethyl)-2-(5- norbornen-2-yl)-1,3-dioxan-5-yl]methanol.

In alternative embodiments, a prepolymer (not shown) may be fabricated by polymerisation of the first cyclic carbonate 22 only.

In alternative embodiments, a prepolymer (not shown) may be fabricated by polymerisation of the second cyclic carbonate 26 only.

In embodiments, polycarbonate 27 may be used as a prepolymer in a resin composition according to the invention.

The prepolymers for use in the resin compositions of the invention may comprise only carbonate linkages, for example, those prepolymers fabricated from either first cyclic carbonate 22 or second cyclic carbonate 26 only. Alternatively, the polycarbonate prepolymers may be further reacted in a chain extension reaction using a diisocyanate (e.g. diisocyanate 28) to produce alternative prepolymers comprising one or more urethane linkages.

## Post Polymerisation Functionalisation

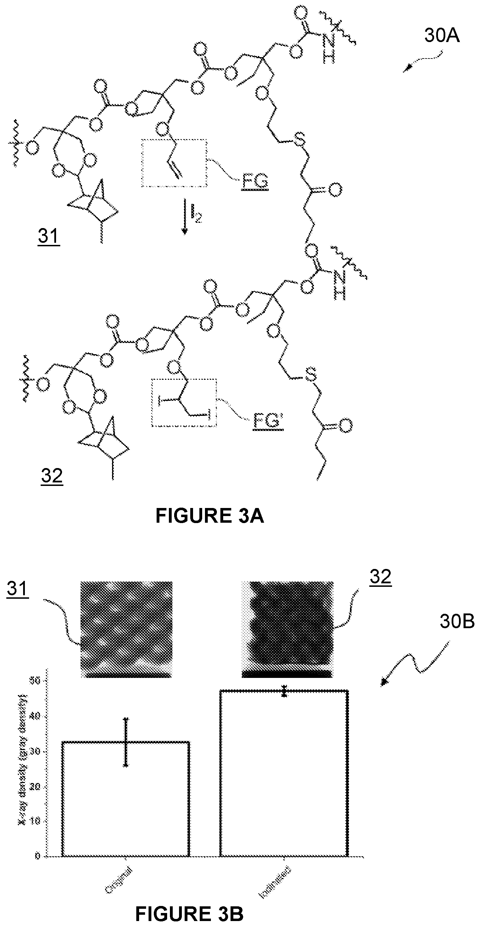

Referring now to Figure 3A, there is shown a schematic reaction 30A of iodination post polymerisation functionalisation of a polymeric material 31, according to an embodiment of the invention. In the schematic reaction 30A, there is shown a polymer 31, and an iodinated polymer 32. Polymer 31 may comprise a functional group FG, which in this an embodiment is an alkene side-chain.

Post-polymerisation, i.e. after the resin composition comprising prepolymer 29 was fabricated into the polymeric material 31 using the stereolithography apparatus, the polymer 31 may undergo reaction with iodine, $I_2$, across the functional group FG to produce an iodinated polymer 32.

Referring also to Figure 3B, there is shown a graph 30B comparing the x-ray density of the polymer 31 and the functionalised polymer 32, according to embodiments of the invention. The graph 30B shows that the iodinated polymer 32 exhibits a greater x-ray density in comparison with the non-iodinated polymer 31. Therefore, the iodinated polymer 32 is visible under clinical imaging such as angiography. This is advantageous for applications wherein the iodinated polymer 32 is a tissue scaffold so that the implant, e.g. implant 10, can be located within the patient, for example, to determine the degradation rate of the iodinated polymer 32 within the implant 10.

In addition, the iodinated polymer 32 may have the following properties in comparison with the non-iodinated polymer 31: (i) the polymer density is increased; (ii) the iodinated polymer 32 is more mechanically stable in comparison with the non-iodinated polymer 31; (iii) reduced rates of mass loss and swelling are observed in comparison with the non-iodinated polymer 31.

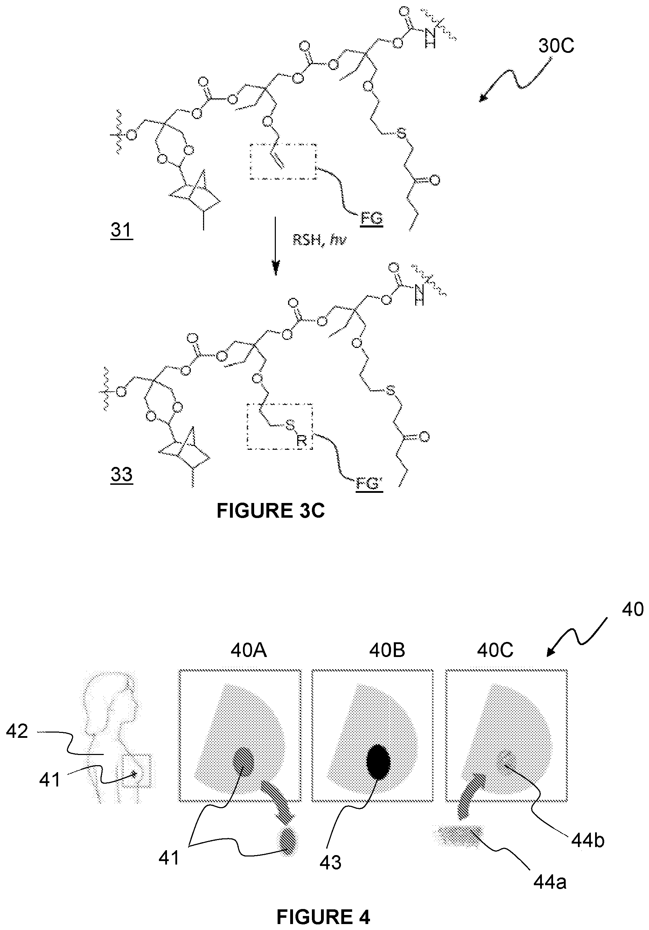

Referring now to Figure 3C, there is shown is a schematic reaction 30C showing alkylation post-polymerisation functionalisation of the polymer 31, according to embodiments of the invention. In the schematic reaction 30C, there is shown the polymer 31, and an alkylated polymer 33. The polymer 31 may comprise a functional group FG, which in this embodiment is an alkene side-chain.

## Therapeutic uses

According to a further aspect of the invention there is provided a method of reconstructing a tissue having a void therein, the method comprising inserting an implant into the void. The implant may be one as described herein.

The method may be for reconstructing body tissue following a surgical procedure on a subject that results in a void in the tissue. For example, the surgical procedure may have removed a tumour in the tissue, e.g. a lumpectomy. Treatment may comprise inserting an implant into the tumour-void. In some embodiments, the method further comprises a surgical procedure that results in a void in the tissue of a subject.

Alternatively, the method may be for reconstructing tissue that is deformed, wounded or that has been subjected to a trauma. The tissue may be soft tissue, such as muscle, fat or fibrous tissue, or it may be hard tissue, such as bone.

In some embodiments, the method comprises inserting the implant in a compressed state. Inserting the implant in a compressed state allows for minimally invasive delivery of the implant. The instrument may be delivered using an instrument, or by hand. For example, the implant may be delivered in a compressed state during key hole surgery.

The method may further comprise, after insertion of the implant, exposing the implant to a stimulus causing it to expand, thereby filling the void.

The present inventors have surprisingly found that the polymeric materials described herein are able to expand to the size and shape of a void. Even under stress, the implant does not change shape or alter the shape of the void. Thus, implants according to the invention are capable of filling, and providing structural support to, a void in tissue (e.g. soft tissue), without exerting pressure on the surrounding tissue.

Depending on the surgery and/or the size of the wound or void, the implant may or may not need compressing prior to insertion.

Exposing the implant to a stimulus may involve contacting the implant with the internal tissue of the void, wherein the heat and/or moisture of the tissue may cause the implant to expand.

The method may further comprise compressing the implant, prior to insertion.

Compressing the implant may comprise heating the implant to a temperature of from 15 to 60 °C, e.g. from 20 to 50 °C, or from 25 to 45 °C. For example, the implant may be heated to a temperature of about 25, 26, 27, 28, 29, 30, 31, 32, 33, 34, 35, 36, 37, 38, 39, 40, 41, 42, 43, 44, or 45 °C.

Compressing the implant may comprise heating the implant to a temperature greater than the glass transition temperature of the polymeric material ($T_g$). For example, the $T_g$ of the cross-linked polymer may be 25, 26, 27, 28, 29, 30, 31, 32, 33, 34, 35, 36, 37, 38, 39, 40, 41, 42, 43, 44, or 45 °C. Compressing the implant may comprise heating the implant to a temperature of at least 25, 26, 27, 28, 29, 30, 31, 32, 33, 34, 35, 36, 37, 38, 39, 40, 41, 42, 43, 44, or 45 °C. Heating may be carried out by any suitable means. Conveniently, the implant may be heated in a water bath, or using a heating gun or laser.

Once heated, the implant may be compressed using a suitable tool, or it may be compressed by hand.

The method may further comprise fixing the size and shape of the implant i.e. fixing the implant in the compressed form. Fixing may be carried out after (e.g. immediately after) compression and prior to expansion/reactivation.

Fixing the shape of the implant may comprise cooling the implant. The implant may be cooled to a temperature which is below the glass transition temperature of the polymeric material. For example, the implant may be cooled to a temperature of less than 35, less than 30 °C, less than 27 °C, less than 25 °C, less than 22 °C, less than 20 °C, less than 18 °C or less than 15 °C. Cooling the polymer or implant may be carried out using a water or an ice bath. The implant may be held in the compressed state during cooling.

Thus, in preferred embodiments, compressing the implant comprises:

l. heating the implant to a temperature greater than the glass transition temperature of the polymeric material;
m. compressing the implant; and
n. fixing the implant in the compressed form, optionally by cooling.

Compressing the implant may comprise reducing the volume of the implant (from its fully expanded state) by at least 10%, at least 20%, at least 30%, at least 40%, at least 50%, or at least 60%. The volume of the implant may be reduced by no more than 90%, no more than 85%, no more than 80% or no more than 70%.

In some embodiments, the method further comprises determining the dimensions of the void. Determining the dimensions of the void may involve scanning the tumour site. The method may comprise selecting an implant which is larger than the minimum dimension of the void.

Implants may be manufactured in a range of sizes, based on the needs of the surgical community, with the option for the surgeon to make minor adjustments (e.g. trim or shape the implant) if required. Thus, in some embodiments the method further comprises modifying the size and/or shape of the implant, prior to insertion. The modification may conveniently be carried out while the implant is in the expanded state, prior to compression.

Alternatively, a bespoke implant may be manufactured to fit the void. In some embodiments, the methods of the invention further comprise fabricating an implant having dimensions which correspond to the internal dimensions of the void. The internal dimensions of the void may be determined (or may have been previously determined) by scanning the void.

In some embodiments, the method further comprises suturing the implant into the void.

The use of the implant of the invention may allow for subsequent monitoring and treatment of a patient, for example by overcoming radiotherapy targeting difficulties encountered after surgical removal of tissue or a tumour mass, e.g. using lumpectomy. For example, a radiopaque implant may improve X-ray targeting by increasing the accuracy during post-operative radiotherapy, thereby improving patient outcomes.

Thus, in some embodiments, the method further comprises determining the presence or location of the implant in the subject.

In some embodiments, the method further comprises administering radiotherapy to the subject, wherein the

radiation is targeted to a location proximal to the implant. Advantageously, the implant may comprise an imaging agent (e.g. a contrast agent) so that the implant is detectable by an imaging technique, such as X-ray. Images (e.g. X-ray images) of the implant can then be used to guide radiotherapy. Since the implant of the invention expands to fill the whole of the tissue void, the whole treatment site will be clearly visible.

In a further aspect, the invention provides a method of identifying a target site for radiotherapy in a subject in need thereof, the method comprising determining the location of an implant in the subject.

The implant of the invention may also enable the healing of the tissue void to be monitored, by monitoring the rate at which the implant is biodegraded and replaced by native tissue. A radiopaque implant, for example an implant comprising an imaging agent, is particularly useful for monitoring healing.

Thus, in a further aspect the invention provides a method of monitoring the healing of a tissue void in a subject, wherein the method comprises:

o. providing an image of the site of the void into which an implant of the invention had been previously inserted; and
p. detecting degradation of the implant.

In some embodiments, the implant comprises a detectable imaging agent, as defined herein. In some embodiments, the implant comprises a radiopaque imaging agent. The image of the void site may be an X-ray image.

In some embodiments, the method further comprises obtaining the image of the site of the void.

Detecting degradation of the implant may comprise comparing the image of the site of the void with a previously obtained image. For example, the image may be compared with an earlier image taken immediately or shortly after insertion of the implant. In another example, the image may be compared with another image taken at least 1 month, at least 3 months, at least 6 months, at least 9 months, at least 12 months, at least 18 months or at least 24 months prior. By comparing the image with an image taken previously, a reduction in the size, mass or volume of the implant can be detected. A reduction in the size, mass or volume of the implant may be indicative of biodegradation of the implant and replacement of the implant by native tissue.

In some embodiments, detecting degradation of the implant comprises using the image of the site of the void to estimate the size, mass or volume of the implant, and comparing the estimated volume with a known size, mass or volume of the implant at the time of insertion.

In some embodiments, monitoring the healing of the tissue void may comprise obtaining a plurality of images of the site of the void. For example, images may be obtained at regular intervals after insertion of the implant (e.g. every 3, 6 or 12 months). Monitoring may be continued until the implant can no longer be detected. The inability to detect the implant may indicate that the implant has completely biodegraded and been replaced by native tissue, thus indicating that healing of the void is complete.

The implant may be one as described herein. In some embodiments, the implant comprises a targeting agent, as defined herein.

In some embodiments, the implant comprises a radioactive material. Optionally, the radioactive material is encapsulated within particles, seeds, ribbons, wires or capsules. The inclusion of a radioactive material in the implant conveniently enables brachytherapy to be delivered.

Thus, in a further aspect the invention provides a method of delivering brachytherapy to a subject in need thereof, the method comprising inserting an implant (e.g. a void occlusion device) into a tissue void in the subject, wherein the implant comprises a radioactive material. The implant may be one as described herein. The implant may be inserted into a void created by the removal of a tumour.

The subject may be a human or a non-human mammal, such as a primate, dog, cat, horse, cow, pig, goat, sheep or rodent.

In some embodiments, the subject is a human. The human may be female. In some embodiments, the subject is suffering from or has previously been diagnosed with cancer, in particular breast cancer or bone cancer. The subject may have undergone surgery to remove a tumour, such as a lumpectomy procedure.

Referring now to Figure 4, there is shown schematically an approach 40 to a treatment procedure using an implant of the invention, wherein the surgical procedure is a lumpectomy.

Referring now to Figure 4, there is shown a schematical approach 40 of the surgical procedure of the invention, wherein the surgical procedure is a lumpectomy.

Firstly, a tumour 41 is isolated and removed from the breast of a patient 42 (Step 40A), in this case the right breast. Removal of the tumour leaves a void 43 (Step 40B). Implant 44 is then inserted into the void 43 (Step 40C) and sutured prior to the incision being closed.

Advantageously, providing an implant that can be delivered into a lumpectomy-cavity eliminates the need for a total mastectomy, reducing both the time in surgery and the recovery period. A mastectomy typically last from 3 to 4 hours and requires a subsequent 3 to 4 days hospitalisation. Whereas, lumpectomy is a 15 to 45 minute same day procedure. Further, the risk of nosocomial infections is reduced as the procedure is less invasive. The surgery also reduces the

number of reconstruction procedures that need to be carried out following conventional lumpectomies, wherein the void is filled with fluid following surgery, which subsequently drains and causes the breast to dimple or deflate.

Prior to removal of the tumour 41, the tumour site may be scanned in order to determine the dimensions of the void 43 so that an implant 44 can be selected that is of the correct shape and size, i.e. satisfies the minimum dimensions of the void 43. Alternatively, a bespoke implant 44 may be manufactured to fit the void 43.

Implant 44 may be compressed by being heated above its $T_g$. The compressed implant 44a may then be cooled in order to modify the shape of the implant. The compressed implant 44a is inserted into the void 43 in the breast. Advantageously, this allows for easier insertion and less invasive delivery of the implant 44.

Once in the void 43 the implant 44 is exposed to an external stimulus, for example moisture from surrounding tissues within the void 43, or additional water applied to the implant 44. On exposure of the stimulus the implant 44 in its first, compressed state 44a expands into a second, expanded state 44b, thereby filling the void 43, i.e. taking on the shape of the void 43 without requiring personalisation, and without compressing the surrounding tissue. Even under stress, implant 44 does not change shape or alter the shape of the void 43. The implant 44 self-fits to the patient, restoring the natural breast cosmetics.

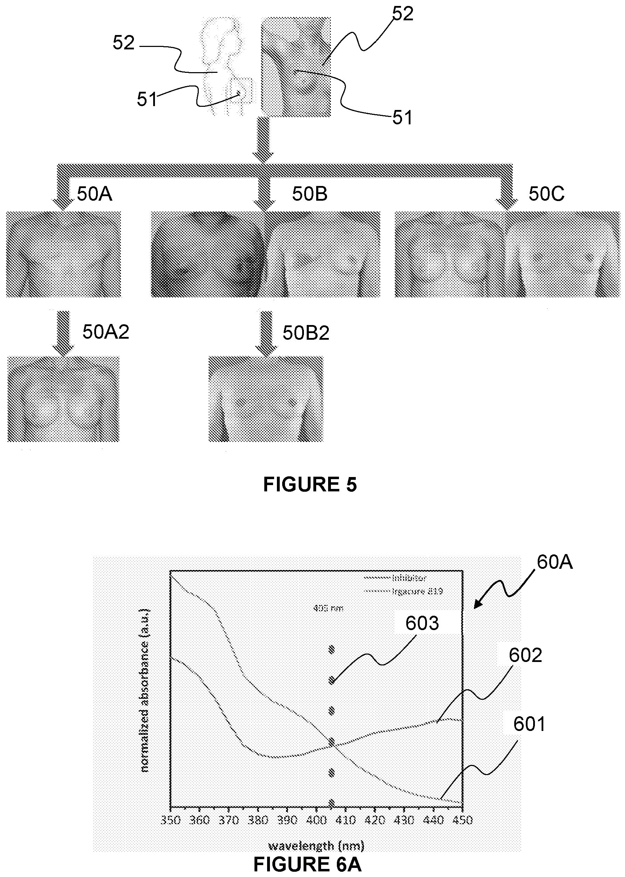

Over time, the implant 44 degrades and is absorbed by the body. Provision of the implant 44 simulates rapid healing of the breast, through gradual erosion and replacement by the patient's own cells/tissue. Further, the risk of collapse or dimpling is reduced or eliminated, allowing for natural breast cosmetics to be maintained by, as shown in Figure 5.

Further, the implant 44 may allow for subsequent monitoring and treatment, overcoming radiotherapy targeting difficulties encountered after surgical removal of tumour mass using lumpectomy. The radiopacity of the implant may improve X-ray targeting by increasing the accuracy during post-operative radiotherapy, improving a patent risk.

Referring to Figure 5, in each case a tumour 51 has been removed from the right breast of a patient 52. Step 50A shows the typical results after a conventional mastectomy, Step 50 B shows the typical results after a conventional lumpectomy and Step 50C shows the results after a lumpectomy according to the embodiment of Figure 4, wherein an implant 44 is inserted within the lumpectomy-cavity, i.e. the void 43 left following removal of the tumour 41/51.

Due to the poor results achieved with conventional mastectomies 50A and lumpectomies 50B, secondary surgery 50A2, 50B2, is often performed in order to attempt to restore the patient's cosmetic appearance. In contrast, a lumpectomy of the invention 50C, where an implant is inserted following tumour removal, does not requires secondary surgery, as the cosmetic appearance following the initial surgery is far superior, with the implant preventing collapse or dimpling of the tumour site.

Advantageously, a treatment involving the insertion of an implant into a lumpectomy-void provides an alternative to mastectomy (total breast removal) and breast reconstruction surgery. Advantageously, conserving the breast shape reduces the psychological impact on the patient. Consequently, this improves a patient's mental health and quality of life (as no subsequent reconstruction is required).

**Example 1: Fabrication of Resin Inks and Photopolymerisation Printing**

**Materials and Methods**

*Instrumentation:* All starting reagents were commercially available (purchased from Sigma-Aldrich unless otherwise stated) and used without further purification. Solvents were of ACS grade or higher. NMR spectra (400 MHz for $^1$H and 125 MHz for $^{13}$C) were recorded on a Bruker 400 spectrometer and processed using MestReNova v9.0.1 (Mestrelab Research, S.L., Santiago de Compostela, Spain). Chemical shifts were referenced to residual solvent peaks at $\delta$ = 7.26 ppm ($^1$H) and $\delta$ = 77.16 ppm ($^{13}$C) for $CDCl_3$ and $\delta$ = 2.50 for ($^1$H) and $\delta$ = 39.52 ppm ($^{13}$C) for $d_6$-DMSO. Size exclusion chromatography (SEC) was performed using an Agilent 1260 Infinity II Multi-Detector GPC/SEC System fitted with RI and ultraviolet (UV) detectors (A = 309 nm) and PLGel 3 $\mu$m (50 $\times$ 7.5 mm) guard column and two PLGel 5 $\mu$m (300 $\times$ 7.5 mm) mixed-C columns with $CHCl_3$ with 5 mM triethylamine as the eluent (flow rate 1 mL/min, 50 °C). A 12-point calibration based on poly(methyl methacrylate) standards (PMMA, Easivial PM, Agilent) was applied for determination of molecular weights and dispersity ($Đ_M$). An Anton Paar rheometer (Anton Paar USA Inc, Ashland, VA, USA) fitted with a detachable photoillumination system with two parallel plates (10 mm disposable aluminum hollow shaft plate, Anton Paar) was used for rheology studies. Uniaxial tensile testing was performed using a Testometric MCT-350 fitted with a 100 kgf load cell (Testometric Company Ltd, Rochdale, United Kingdom). Dynamic mechanical analysis was performed using a Mettler-Toledo TT-DMA system (Mettler-Toledo AG, Schwerzenbach, Switzerland) fitted with an equilibrating water bath and water circulator, and samples analyzed using Mettler-Toledo STARe v.10.00 software. 3D printing scaffolds and templates were processed using Solidworks (Dassault Systemes, Vélizy-Villacoublay, France) and printed using a custom digital light processing system that has been previously reported.[58] Micro-computed tomography analysis was performed using a Skyscan 1172 MicroCT (e2v technologies plc, Chelmsford, UK) at an isotropic pixel size of 7-13 $\mu$m, a camera exposure time of 500 ms, a rotation step of 0.4°, frame averaging of 5 and medium filtering with a flat field correction. Image

reconstruction was performed using a NRecon 1.6.2 (SkyScan, e2v technologies plc, Chelmsford, UK).

*Synthesis of TMPAC monomer:* Trimethylolpropane allyl ether (100.0 g, 573.7 mmol) was added to a round bottom flask with 200 mL tetrahydrofuran (THF), and cooled to 0 °C for 1 h. Ethyl chloroformate (124.5 g, 1.1 mol) was added as a single volume to the solution and allowed to again cool to 0 °C for 15 min. Triethylamine (116.2 g, 1.1 mol) was added dropwise over the course of 1 h, at which time the solution was allowed to slowly return to ambient temperature. The precipitate was filtered off and the solute concentrated to a slightly yellow oil, and dissolved in ethyl acetate. The organic layer was washed twice with 1 M HCl and once with brine, and concentrated to a colorless, slightly viscous oil. The oil was distilled to achieve cyclic TMPAC (98.8 g, 493.8 mmol, 86% yield). Characterization matched previously reported materials. $^{1}H$ NMR ($CDCl_3$, 400 MHz): $\delta$ = 0.94 (t, $^{3}J_{H-H}$ = 7.6 Hz), 1.55 (q, 2 H, $^{3}J_{H-H}$ = 7.6 Hz), 3.47 (s, 2 H), 3.88-4.05 (m, 2 H), 4.23 (d, $^{3}J_{H-H}$ = 10.1 Hz, 2 H), 4.52 (d, $^{3}J_{H-H}$ = 10.1 Hz, 2 H), 5.21-5.42 (m, 2 H), 5.78-5.90 (m, 1 H) ppm. $^{13}C$ NMR ($CDCl_3$, 125 MHz): $\delta$ = 9.1, 23.3, 37.1, 68.2, 72.4, 72.9, 117.0, 134.4, 148.9 ppm.

*Synthesis of NTC monomer:* Pentaerythritol (40.9 g, 300.6 mmol) was added to a round bottom flask and suspended in 500 mL of deionised water heated to 80 °C. The mixture was stirred until the solids had dissolved and was then cooled to 20 °C. 2 drops of concentrated HCl (~500 μL) was added, followed by 5-norbornene-2-carboxaldehyde (30.5 g, 253.8 mmol), after which the solution was stirred for 8 h. The product, an orange precipitate, was isolated using vacuum filtration and recrystallized from hot toluene/IPA (80/20) as white crystals (NHD). NHD (17.0 g, 71.0 mmol) was dissolved in 400 mL THF in a round bottom flask and cooled to 0 °C, at which point ethyl chloroformate (20.4 mL, 212 mmol) was added as a single volume and allowed to cool again to 0 °C. Triethylamine (29.5 mL, 212 mmol) was added dropwise over 1 h, and the reaction was allowed to come to 20 °C before stirring for 12 h. The precipitate was filtered and the solute concentrated to yield white crystals. The white crystals were recrystallized in hot cyclohexane/THF (90/10) (15.4 g, 58.7 mmol, 71%). Characterization matched previously reported materials.[60] $^{1}H$ NMR (DMSO-$d_6$, 400 MHz,): $\delta$ = 6.17 (q, 1 H, $^{3}J_{H-H}$ = 5.7, 3.0 Hz), 5.93 (q, 1H, $^{3}J_{H-H}$ = 5.7, 2.8 Hz), 4.51 (s, 2 H), 4.06 (s, 2 H), 3.89-3.83 (m, 3 H), 3.61-3.58 (m, 2 H), 2.85 (s, 1 H), 2.78 (s, 1 H), 2.22 (m, 1 H, $^{3}J_{H-H}$ = 12.8, 8.6, 3.9 Hz), 1.75 (m, 1H, $^{3}J_{H-H}$ = 12.8, 9.3, 3.8 Hz), 1.31-1.17 (m, 2 H), 0.74 (m, 1 H, $^{3}J_{H-H}$ = 11.9, 4.1, 2.6 Hz). $^{13}C$ NMR (DMSO-$d_6$, 125 MHz;): $\delta$ = 28.2, 30.7, 41.2, 43.0, 43.1, 48.9, 67.9, 68.1, 70.7, 106.0, 133.0, 137.6, 161.9.

*Synthesis of aliphatic polycarbonate:* Ring opening polymerization of the cyclic monomers was used to obtain oligomers. To an open round bottom flask, $CHCl_3$ and cyclic monomer(s) were added followed by 1,8-diazabicyclo[5.4.0] undec-7-ene (DBU). For PolyTMPAC, TMPAC (100 g, 500.0 mmol) was dissolved in 100 mL $CHCl_3$. DBU (1.44 g, 9.5 mmol) and water (150 μL, 8.3 mmol) were added as a single unit. The resulting solution was stirred for 24 h at 20 °C, after which the DBU was quenched with the addition of Amberlyst A15 $H^+$ acidic resin, precipitated into ice cold hexanes, and was then filtered through a silica plug in ethyl acetate. The solution was concentrated *in vacuo* to yield a viscous, colorless liquid (96.2 g, 96%). $^{1}H$ NMR (DMSO-$d_6$, 400 MHz,): $\delta$ = 0.82 (t, $^{3}J_{H-H}$ = 7.6 Hz, 3H), 1.45 (d, $^{3}J_{H-H}$ = 9.4 Hz, 2H), 3.32 (s, 2H), 3.87 (dd, $^{3}J_{H-H}$ = 5.4 Hz $^{3}J_{H-H}$ = 1.8 Hz, 2H), 4.04-4.21 (m, 4H), 5.11-5.32 (m, 2H), 5.79-5.93 (m, 1H), 6.88 (s, 1H). $^{13}C$ NMR (DMSO-$d_6$, 125 MHz;): $\delta$ = 7.3, 14.1, 20.7, 22.4, 41.5, 43.3, 61.6, 69.1, 69.5, 70.2, 72.3, 115.8, 135.3, 154.6. SEC ($CHCl_3$) $M_n$: 6. kDa, $Đ_M$ = 1.2.

*Synthesis of aliphatic poly(carbonate urethane):* In a representative synthesis of the poly((TMPAC-co- hexamethylene diurethane), PolyTMPAC (2 kDa, 5.0 g, 2.5 mmol) was dissolved in a round bottom flask containing dry THF at 60 °C under $N_2$, to which hexamethylene diisocyanate (HDI) (1.0 g, 6.0 mmol) was added. The mixture was allowed to stir for 48 h, during which time the viscosity visually increased dramatically. At 48 h, the temperature was increased to 80 °C and allowed to stir for 12 h, at which time the entire solution was added to 50 mL MeOH. The solution was concentrated, washed with 1 M HCl twice and once with saturated brine solution, and collected as a highly viscous, transparent oil (5.94 g, 99%). $^{1}H$ NMR (DMSO-$d_6$, 400 MHz,): $\delta$ = 0.82 (t, $^{3}J_{H-H}$ = 9.0, 6.0 Hz, 3H), 1.33 (m, $^{3}J_{H-H}$ = 7.6 Hz, 2H), 1.55 (q, $^{3}J_{H-H}$ = 7.5 Hz, 2H), 1.76 (s, 2H), 3.27 (d, $^{3}J_{H-H}$ = 9.0 Hz, 2H), 3.46 (s, 2H), 3.64 (s, 2H), 3.89 (s, 2H), 4.07 (dd, $^{3}J_{H-H}$ = 9.0 Hz, $^{3}J_{H-H}$ = 4.4 Hz, 2H), 5.09-5.20 (m, 2H), 5.78-5.85 (m, 1H), 6.88 (s, 1H). $^{13}C$ NMR (DMSO-$d_6$, 125 MHz;): $\delta$ = 7.3, 22.5, 23.2, 25.5, 25.8, 30.9, 35.3, 42.7, 68.1, 72.7, 77.2, 116.8, 117.2, 133.9, 134.4, 148.5, 155.3, 155.7. SEC ($CHCl_3$) $M_n$: 6.2 kDa, $Đ_M$ = 1.6.

*Synthesis of isophorone di(allyl urethane):* Isophorone diisocyanate (55.53g, 0.250 moles) was added by canula transfer to a round bottom flask (dried 120 °C overnight and sealed) followed by dry 200 mL THF. Freshly distilled allyl alcohol (30.64 g, 0.528 moles), stored over molecular sieves, was added dropwise to the solution while stirring at 300 rpm. Upon complete transfer of the allyl alcohol, the reaction was heated to 50 °C and held isothermally for 24 h, at which point residual diisocyanate was quenched with water (at 50 °C). Crude urethane was obtained after dissolving the reaction mixture in ethyl acetate, washing with 1M HCl (3 washes) and brine (1 wash) and concentrating the product. A viscous clear oil was collected after column chromatography (90:10 EtOAc:Hexane) and concentrated *in vacuo* to yield a colorless oil (83.2 g, 246.0 mmol, 96.0 %). Characterization matched previously reported materials.[61] $^{1}H$ NMR ($CDCl_3$, 400 MHz): $\delta$ = 0.83-0.92 (m, 6H), 1.05 (s, 3H), 1.17-1.21 (d, $^{3}J_{H-H}$ = 9.0 Hz, 2H), 1.36-1.40 (d, $^{3}J_{H-H}$ = 12.0 Hz, 2H), 1.67-1.74 (t, $^{3}J_{H-H}$ = 9.0 Hz, 2H), 1.85-1.88 (d, $^{3}J_{H-H}$ = 9.0 Hz, 2H), 2.91-2.92 (d, $^{3}J_{H-H}$ = 3.0 Hz, 2H), 3.79-3.81 (m, 1H), 4.53-4.55 (d, $^{3}J_{H-H}$ = 9.0 Hz 2H), 4.84 (s, 1H), 5.18-5.31 (m, 2H), 5.85-5.94 (m, 1H). $^{13}C$ NMR ($CDCl_3$, 125 MHz): $\delta$ = 23.3, 27.7, 29.8, 32.0, 35.1, 36.5, 412.0, 44.8, 46.4, 47.2, 55.0, 65.7, 117.8, 133.0, 155.6, 156.8 ppm.

*Formulation of Poly(TMPAC) resins.* Stoichiometric amounts of PolyTMPAC and crosslinker were added to a vial,

along with the 4 arm tetrathiol (pentaerythritol tetrakis(3-mercaptopropionate) (PETMP)) in stoichiometric amounts. As an example, the PolyTMPAC resin consisted of isophorone di(allyl urethane) (13.78 g, 40.7 mmol), PolyTMPAC (15.28 g, 7.6 mmol), 1,3,5-triallyl-1,3,5-triazine-2,4,6(1H,3H,5H)-trione as a reactive diluent (14.65 g, 58.7 mmol), PETMP (24.41 g, 53.2 mmol), and of propylene carbonate as an unreactive diluent (16.54 g, 162.1 mmol) mixed together for 8 h at ambient conditions. To this was added Irgacure 819 (photoinitiator, 0.82 g, 1 wt%), and paprika extract (photoinhibitor, 0.50 g, 0.75 wt%) in a dark room with little ambient light, followed by 1 h of stirring. After homogenization of the resin, the resin was placed in a brown glass container and stored at room temperature in the dark.

*Spectroscopic Analysis of Thiol-ene Crosslinking.* Conversion of alkenes in oligomeric and monomeric reactive components in the presence of PETMP with 1% wt photoinitiator and no inhibitor were performed to study crosslinking kinetics. Experiments were performed in 0.5 mL $CDCl_3$ at ambient conditions, exposed to $\lambda$ = 340 to 430 nm light for discrete timepoints prior to storage in brown glass vials.

*Photorheology.* Crosslinking kinetics of resin samples were examined as a function of gelation time by measuring the dampening or phase ratio (tan $\delta$), storage moduli, loss moduli, complex viscosity, and film thickness during photorheology. Resin samples were sheared between two parallel plates, one made of glass and transparent, at 1 Hz for 50 sec without irradiation. After this time, the resins were irradiated with $\lambda$ = 430-520 nm light and measurements were taken every 0.2 s over the course of 2 min. The inflection points of the moduli plots, and the peak tan $\delta$ values, were used to determine the time to gelation of the resin. Sample shrinkage was measured by measuring the distance between the plates at the same sampling rate as the other metrics.

**Results**

In order to achieve the degradable polymer backbones without acidic degradation, and while maintaining good control over the synthesis, organocatalytic ring opening polymerization (ROP) of aliphatic cyclic carbonates was selected. This process yielded homo- and co-oligocarbonates from allyl- and norbornene-containing monomers (TMPAC and NTC respectively) with a targeted number-average molar mass (*Mn*) ca. 2 kDa and a dispersity, $Đ_M$, of 1.1. Analysis by $^1$H nuclear magnetic resonance (NMR) and Fourier-transformed infrared (FT-IR) spectroscopy confirmed the presence of carbonyl, hydroxyl, and alkene groups which, in addition to size exclusion chromatography (SEC) analysis, confirm oligomer synthesis. Physically, oligomers with higher NTC content resulted in solid polymers, while polyTMPAC homopolymer and those with high TMPAC contents were slightly viscous oils.

In order to achieve photocrosslinked materials, the miscibility of the oligomers was examined using chain extension with aliphatic diisocyanates to yield poly(carbonate urethane)s (PCUs), or by the addition of urethane-containing reactive diluents. Additionally, the oligomers were diluted and solubilized into PETMP to reduce viscosity below 10 Pa·s and create resins suitable for photo-initiated crosslinking and 3D printing. The chain extended PCUs displayed viscosities more than an order of magnitude higher than the polycarbonate resins. Focus remains on the urethane-containing reactive diluents for the majority of subsequent testing.

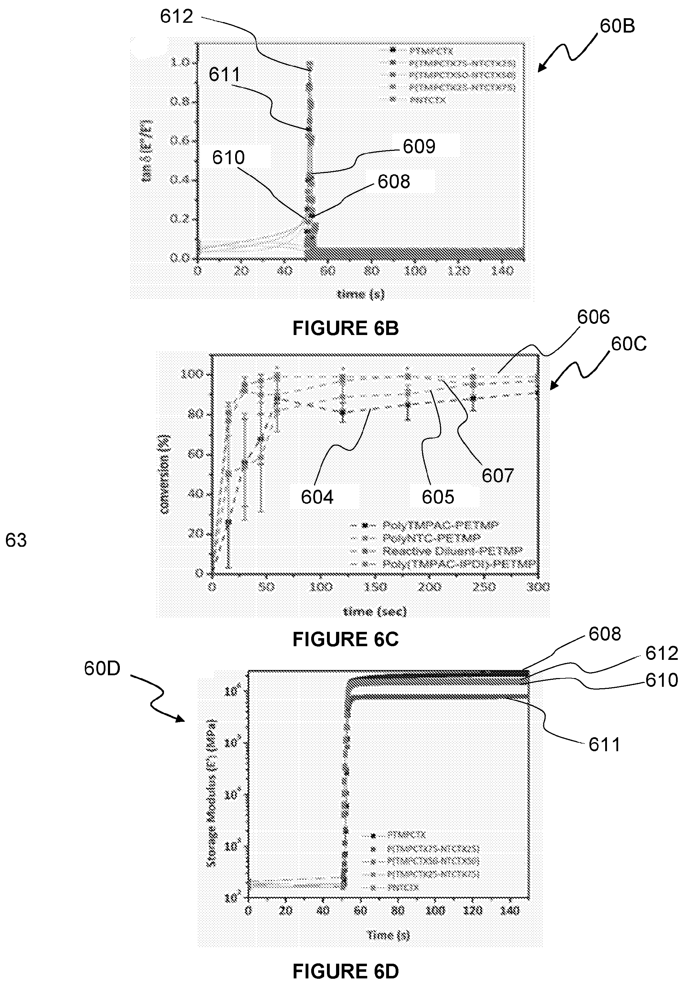

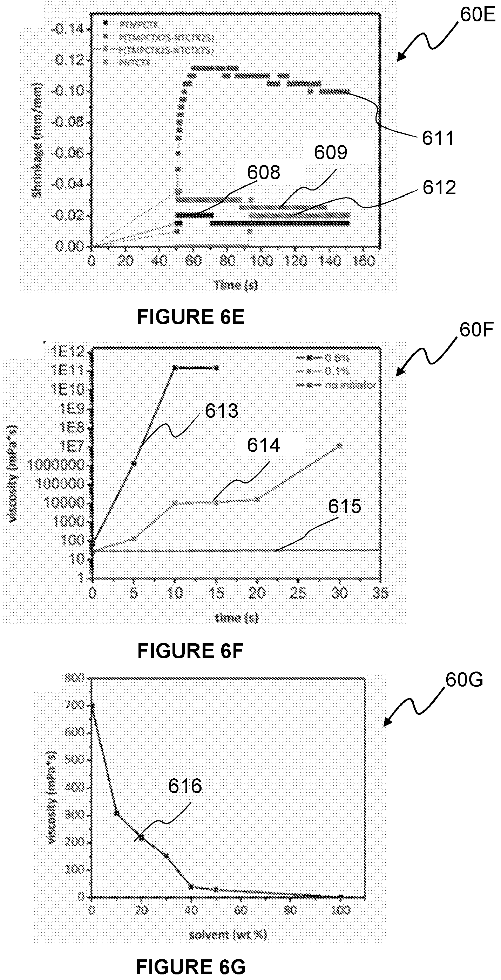

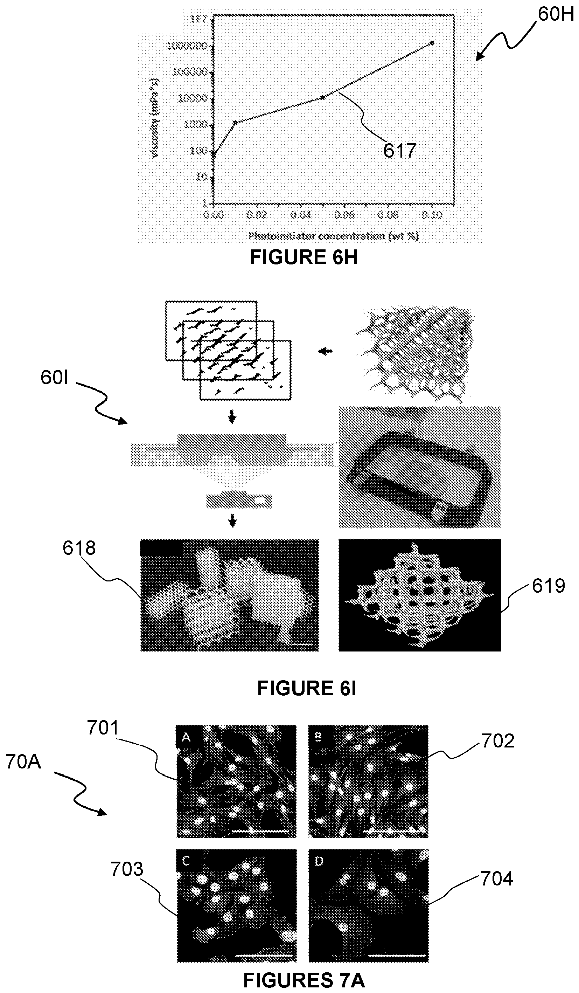

Referring now to Figure 6A, a photoinitiator active at $\lambda$ = 405 nm (Irgacure 819, 601) and a paprika extract-derived photoinhibitor (602) with competitive absorbance (60A) in the same region (Figure 6A) resulted in orange, slightly viscous resin inks (in batches up to 150 g) which when processed, allow a high degree of spatial control without competitive absorbance by the polymeric resin components. The liquid resins rapidly undergo phase transitions to gelled solids upon irradiation in the visible light spectrum (Amax = 405 nm, 603).

Photo rheological analysis revealed a peak loss factor ratio at 2 s after irradiation and a dramatic increase in both storage modulus and complex viscosity, from 179.6 $\pm$ 17.5 Pa to 1.5 $\pm$ 0.4 MPa and 3.1 $\pm$ 0.1 Pa·s to 23.1 $\pm$ 8.3 MPa·s respectively, followed by a plateau even upon further irradiation (Figure 6B).

Referring to Figures 6B to 6H, the photorheology of the resins with 1% wt photoinitiator and 1% photoinhibitor are shown. Resin samples were sheared between two parallel plates, one made of glass and transparent, at 1 Hz for 50 s without irradiation at ambient conditions. After this time, the light source was switched on and measurements were taken every 0.2 s over the course of 2 min. Loss factor (tan $\delta$) (60B), conversion % (60C of polyTMPAC-PETMP 604, polyNTC-PETMP 605, reactive diluent-PETMP 606 and poly(TMPAC-IPID)-PETMP 607 monomers) and storage moduli (60D) plots for resin compositions are displayed over time, accompanied by resin shrinkage over the course of film curing (60E).

$^1$H NMR spectroscopic analysis of oligomers and model compounds in the presence of the PETMP crosslinker further confirms the rapid, efficient thiol-ene crosslinking within 30 s of exposure resulting in rapid consumption of the allyl and thiol groups and ultimately, gel formation. The chemical flexibility of the resin system enabled polyTMPAC to be used to produce PTMPCTX 608 (polyTMPAC-derived thioether crosslinked) scaffolds, while polyNTC was used to produce PNTCTX 609 (polyNTC-derived thioether crosslinked) scaffolds, where a 50:50 copolymer of the materials would be P(TMPCTX50-NTCTX50) 610, a 75:25 copolymer of the materials would be P(TMPCTX75-NTCTX25) 611 and a 25:75 copolymer of the materials would be P(TMPCTX25-NTCTX75) 612.

The rate of crosslinking over time (with initiator concentrations of 0.5% 613, 0.1% 614 and 0% 615, viscosity vs diluent concentration 616, and viscosity vs photoinitiator concentration 617 are plotted (60F, 60G, 60H, respectively) for

PTMPCTX (polyTMPAC-derived thioether crosslinked) resins.

Referring to Figure 6I, using digital light processing (DLP, 60I), a stereolithographic-type process, all compositions could be used to produce porous scaffolds (pictograph 618 and micro CT images 619), with potential for void filling devices, without the need for additional processing to remove foam cell membranes or additives typically found in porous biomaterials. To demonstrate the ability to print a range of scaffold geometries, scaffolds were printed with pore sizes ranging from 200 to 1500 μm, and surface areas of between 1 and 3 $cm^2$. Analysis of the resulting scaffolds by μCT revealed that the measured pore size values match the theoretical pore sizes calculated from the theoretical porous structure renderings within 5% error (Figure 6E).

**Example 2: Cellular Response to Carbonate Based Materials**

**Materials and Methods**

*Cytocompatibility and Cellular Analysis:* Samples for cell culture studies (n = 4) were prepared by spin coating a solution of 0.4 wt% polymer in $CHCl_3$ on a glass coverslip (1 min at 1000 rpm). Spin-coated glass coverslips were then placed into 12 well plates for ethanol sterilization. NOR-10 (murine fibroblasts), Hs 792 (human fibroblasts), IC21 (murine macrophages), and D16 (murine adipocytes) cell lines were purchased from ATCC UK and cultured in DMEM (NOR-10 and Hs 792), RPMI-1640 (IC21), and DMEM/F12 (D16) media supplemented with 10% FBS (20% for NOR-10) and 1% pen/strep, at 37 °C and 5% $CO_2$. 1% L-Alanyl-L-Glutamine was added in DMEM/F12 medium.

*Cell Proliferation:* Cell proliferation assay was performed on spin-coated glass slides by seeding the above cell lines (n = 4, 2000 cells $cm^{-2}$) and measuring metabolic activity at selected time points (24 h, 3, 7, and 14 days of culture). Cell proliferation was evaluated by using a PrestoBlue® metabolic assay following the supplier's instructions. Briefly, after removing the medium, 1 mL of PrestoBlue® solution (10% in cell culture medium) was added to each well, followed by incubation at 37 °C for 1-4 h. Sample fluorescence was read when the fluorescence from the standard curve gave a linear fit. 100 μL of solution was taken from each well and placed in triplicate into a 96 well plate. The fluorescence intensity (FI) was detected in a BioTek® SynergyTM MX Microplate Reader at wavelengths of 590 nm for excitation and 610 nm for emission.

*Cell Spreading:* Cells were seeded on spin-coated coverslips (n = 4) at 4000 cells·$cm^{-2}$. After 72 h, cells were fixed using a 4% paraformaldehyde solution for 10 min, permeabilized using 0.5% Triton X-100 in cytoskeleton stabilization (CS) buffer (0.1 M PIPES, 1 mM EGTA, and 4% (w/v) 8000 MW polyethylene glycol) at 37 °C for 10 min, rinsed thrice for 5 min each in CS buffer, and incubated in 0.1% sodium borohydride in PBS at ambient temperature for 10 min to quench aldehyde autofluorescence. Samples were then blocked in 5% donkey serum for 20 min at 37 °C and incubated overnight at 4 °C with mouse primary anti-vinculin antibody (1:100). Samples were then washed three times with 1% donkey serum for 5 min each, and then incubated with Alexa Fluor 647 Phalloidin for cytoskeleton staining (1:200) for 1 h followed by Alexa Fluor® IgG-594 secondary antibody (donkey anti-mouse, 1:100). DAPI was used to stain the cell nuclei. Cells were imaged with a FV3000 Olympus confocal fluorescence microscope using 350 nm, 594 nm, and 633 nm excitation filters and a 20 or 40x oil immersion objectives.

*3D cell experiments:* 3D printed scaffolds were sterilized by immersion in 70% ethanol, placed in 24 well plates, and incubated for 24 h in cell culture medium at 37 °C, 5% $CO_2$. The medium was then removed and cells (100,000 in 20 μL of medium) were seeded on top of the scaffolds (n = 3) and incubated at 37 °C, 5% $CO_2$ for 3 h. After this time, 2 mL of culture medium was added and the cells were incubated again at 37 °C, 5% $CO_2$ for the selected time points (24 h, 3 days, 7 days). A live/dead assay (Invitrogen) was performed at each of the selected time points. Briefly, scaffolds were washed with PBS (3 × 2 mL) and incubated with a calcein/ethidium homodimer solution at 25 °C for 20 min, following the supplier's instructions. Scaffolds were then washed with PBS (3 × 2 mL) and placed on a microscope slide for fluorescent imaging. Cells were imaged with a FV3000 Olympus confocal fluorescence microscope using 488 nm and 594 nm excitation filters and a 4x air objective. Image J was used for analysis.

*Surgical Procedure:* Experiments were performed in accordance with the European Commission Directive 2010/63/EU (European Convention for the Protection of Vertebrate Animals used for Experimental and Other Scientific Purposes) and the United Kingdom Home Office (Scientific Procedures) Act (1986) with project approval from the institutional animal welfare and ethical review body (AWERB). Anaesthesia was induced in adult male Sprague Dawley rats (200-300 g) with isofluorane (2-4%; Piramal Healthcare) in pure oxygen (BOC). Animals were placed prone onto a thermocoupled heating pad (TCAT 2-LV; Physitemp), and body temperature was maintained at 36.7 °C. The experimental material and control material (PLLA) were implanted over either the spinotrapezius or lateral aspect of the external obliques. Following an incision of ~3 cm, the skin was separated from the muscle with large forceps, and any excess fat was removed. The implants were tunnelled under the skin and placed in direct contact with the muscle, at sites distal to the incision. The order of the implants was randomised, but constrained so that each implant appeared in each location bilaterally at least once. The wounds were sealed with a subcuticular figure of 8 purse string suture with a set-back buried knot using 3-0 vicryl rapide suture (Ethicon). The surgical procedure was performed under the strictest of aseptic

conditions with the aid of a non-sterile assistant. Post-surgical analgesia was administered, and rats were placed into clean cages with food and water ad *libitum.*

**Results**

Cytocompatibility screening was performed using 2D surfaces, in order to assess compositional factors prior to final scaffold development, and in 3D scaffolds as a more realistic model. No significant differences were found regarding proliferation or morphology when assessed over a 7-day period (murine fibroblasts, murine adipocytes, murine macrophages, and human fibroblasts) in both direct and indirect contact assays based upon ISO 10993 protocols. All cell types, including macrophages, adipocytes and fibroblasts (murine and human) are representative of those found in native adipose tissue, displayed good cell spreading and adhesion (Figure 7A). No statistical significance was found for live-dead ratios or proliferation rates over 7 days based on composition for both assay types.

Figure 7A shows representative images 70A of adipocytes (701 and 702) and fibroblasts (703 and 704) for PTMPTCX (701 and 703) and PNTCTX scaffolds (702 and 704). (Scale bar = 10 $\mu$m)

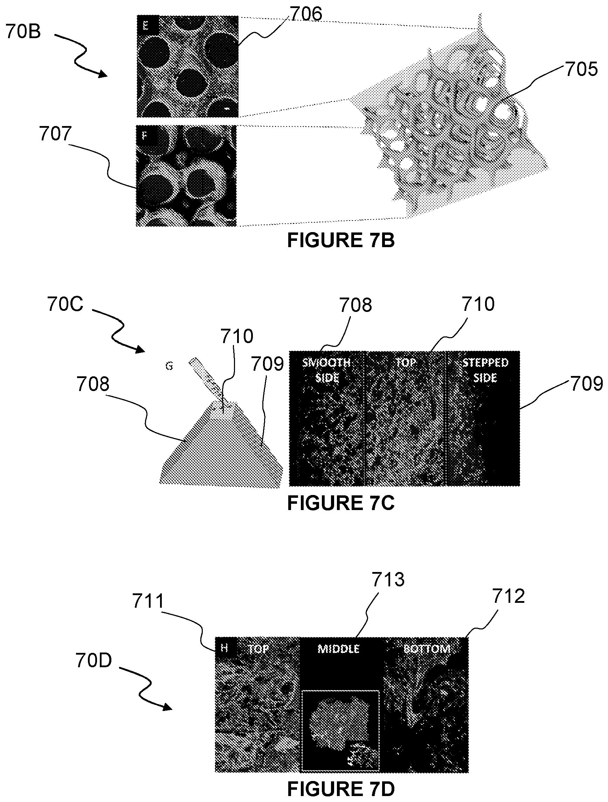

In 3D culture, cells were found to proliferate throughout the entire scaffold for pore sizes between 250 to 1500 $\mu$m, the preferred range for tissue scaffolds to allow for nutrient diffusion and proliferation into a material (Figure 7B).

Figure 7B shows confocal images of adipocytes 70B on 3D PTMPTCX scaffolds 705 at the top 706 and bottom 707 after 7 days proliferation. (Scale bar = 100 $\mu$m)

In order to determine if the step-layer structure that results from the 3D printing process was partly responsible for the high cytocompatibility of the scaffolds, PTMPCTX-based materials were 3D printed into pyramidal structures 70C with a glass-cast smooth side 708 opposite a stair-step side 709, joined by a flat-top 710 for cell seeding (Figure 7C). On the pyramidal scaffolds, fibroblasts were found to proliferate equally down both the stair steps as the smooth surface, which indicates that the excellent cytocompatibility is a result of the polycarbonate chemistry rather than surface morphology. Corresponding cell images from both surfaces overlaid to display cell migration after 7 days (Figure 7G), display no differences between surface morphology and cellular proliferation.

To allow further examination of the structural versatility allowed by this approach, materials were also foamed using a modified gas-blowing procedure to produce porous scaffolds (Figure 7H). The gas-blown scaffolds displayed a high degree of cytocompatibility over 7 days, without the 3D proliferation that was observed in the 3D printed structures. In the foams, fibroblasts proliferated along the top outer layers of the foam with some also being found along the bottom surface but critically, in contrast the 3D printed structures, no cellular infiltration into the centre of the foams were observed in this time frame. This most likely is a consequence of the very fine pore structure and limited interconnectivity between pores limiting diffusion and preventing cellular infiltration beyond the initial layer of pores. Representative images of cellular proliferation throughout PTMPTCTX foam with images 70D taken at the top of the scaffold 711 (where cells were seeded), bottom of the scaffold 712, and from the middle of the scaffold 713 after the same time, inset $\mu$CT of foams, are shown in Figure 7D.

**Example 3: Scaffold Thermomechanical Behaviours**

**Materials and Methods**

*Mechanical Testing.* Printed dogbones (modified ASTM Type IV) were examined using uniaxial tensile testing at ambient temperature. Samples were placed in the tension clamps and allowed to vibrationally equilibrate for 10 min, at which point each sample was extended at 5 mm·min$^{-1}$ until failure. Seven samples were run per composition.

*Dynamic Mechanical Analysis:* Rectangular dynamic mechanical analysis (DMA) samples were prepared *via* 3D printing sample bars (2.0 cm $\times$ 0.5 cm $\times$ 0.2 cm). Samples were analyzed in tension mode using autotension mode, with a frequency of 1 Hz, a preload force of 1 N, and a static force of 0.1 N. Three samples were used in each analysis.

*Thermal analysis:* Thermal sweeps were conducted at 2 °C·min$^{-1}$, starting at -30 °C and ending at 200 °C before cooling to ambient conditions at an average initial rate of 10 °C·min$^{-1}$ to 60 °C, followed by 2 °C·min$^{-1}$ to room temperature, as which point the scaffold was cycled again for 15 cycles. The peak ratio between the loss and storage moduli (*E"/E', tan* $\delta$) was defined as the $T_g$. This method was used to determine curing kinetics of the films, as well.

*Polymer relaxation kinetics:* Relaxation kinetics studies of the printed scaffolds were conducted using submersion DMA at 37 °C in phosphate buffered saline (PBS) solution, in oscillation mode. Scaffolds (1 cm$^3$) were placed in compression and deformed 10 $\mu$m, 1 Hz with a preload of 0.1 N at ambient conditions for approximately 60 sec. At this time, the scaffold was then immersed in the PBS solution and held isothermally as the same load was applied for 60 min. Storage moduli and tan $\delta$ values were recorded as a function of time to determine the behavior of the polymer during initial submersion/introduction to biologically-mimicking conditions. Expansion forces were measured using the same method in creep mode.

**Results**

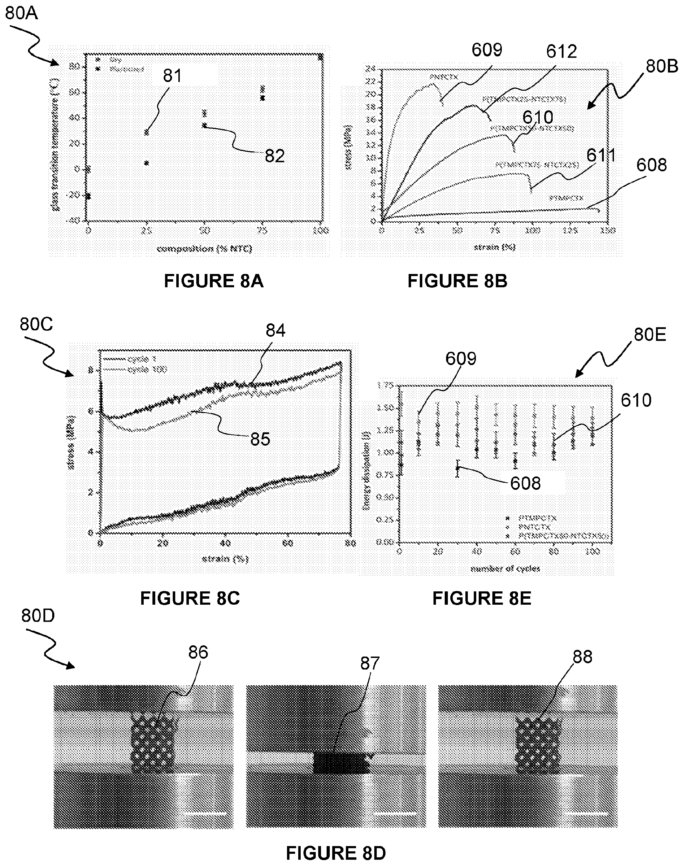

The synthetic versatility of the resin formulation allows the thermomechanical properties of the resultant photocured materials to be tuned with respect to stiffness and stimuli-response temperature (and in turn shape memory response temperature or plasticization *in vivo*). The carbonate monomer ratio and the presence of urethane linkages were used to tune the glass transition temperature across a range of more than 100 °C in both dry and solvated conditions (Figure 8A and Table 1).

Figure 8A shows the relationship 80A between $T_g$ and NTC concentration in the printed polycarbonate materials (dry 81 and plasticized 82) as determined from phase transitions examined using DMA compression.

Table 1. Thermomechanical properties of 3D printed polycarbonates. (n=5)

| Composition | Glass Transition Temperature ($T_g$) (°C) | Plasticized Glass Transition Temperature (°C) | Compressive Modulus (MPa) | Elastic Modulus (MPa) | Strain at Break (%) | Ultimate Tensile Strength (MPa) | Toughness (MPa/m$^2$) |
|---|---|---|---|---|---|---|---|
| **PTMPCTX** | 0.3 ± 2.3 | -20.6 ± 1.9 | 1.1 ± 0.5 | 15.2 ± 7.6 | 144.1 ± 33.2 | 2.1 ± 0.1 | 213.3 ± 51.4 |
| **P(TMPTCTX75-NTCTX25)** | 29.2 ± 1.9 | 5.3 ± 1.2 | 3.0 ± 1.2 | 36.2 ± 5.5 | 99.0 ± 22.4 | 7.6 ± ± 1.4 | 514.6 ± 97.1 |
| **P(TMPTCTX50-NTCTX50)** | 43.9 ± 2.4 | 34.5 ± 1.4 | 12.2 ± 5.0 | 196.5 ± 12.8 | 87.8 ± 21.1 | 13.7 ± 4.9 | 821.7 ± 157.3 |
| **P(TMPTCTX25-NTCTX75)** | 62.8 ± 2.3 | 55.8 ± 1.7 | 9.3 ± 2.0 | 122.6 ± 18.1 | 67.2 ± 18.7 | 18.3 ± 3.8 | 906.8 ± 192.8 |
| **PNTCTX** | 88.2 ± 1.1 | 87.1 ± 1.1 | 12.3 ± 4.8 | 776.0 ± 59.1 | 40.6 ± 13.5 | 22 ± 4.1 | 723.5 ± 167.9 |

It was found that the same $T_g$ increase observed with PCUs that are produced through chain extension of polycarbonates could be achieved through incorporation of the isophorone-derived reactive diluent. Higher NTC content in the material increased the dry and plasticized $T_g$s and also decreased the extent of polymer chain relaxation, as determined thermomechanically through immersion testing in phosphate buffered saline (PBS, pH = 7.4) of cast films examined with dynamic mechanical analysis (DMA). Similarly, the mechanical performance of the materials could also be controlled over a wide range by modulation of the resin composition (Figure 8B, Table 1). PNTCTX 609, the highest $T_g$ composition, displayed a tensile elastic modulus of nearly 660 MPa and ultimate tensile strength of approximately 22 MPa at 32% strain, after which the material fractured. In contrast, the PTMPCTX material 608 displayed ca 140% strain to failure, with an elastic modulus of nearly 15.2 MPa and ultimate strength of 2.1 MPa (80B) showing that the materials can be tuned to a potentially broad set of application areas with differing mechanical demands. The materials were all found to be fully elastic until failure at both room temperature and when immersed in PBS at 37 °C.

Figure 8C shows the representative cyclic compression behavior 80C of printed porous PTMPCTX scaffolds 608 in 37 °C PBS (following a single cycle 84 and after 100 cycles 85). Representative images of the PTMPTCX scaffold deformation 80D at 25 °C are shown before loading 86, at 70% strain 87, and after the load is removed 88. Scale bar = 1 cm. Corresponding energy absorption for 100 cycles in alginate gels examined at 37 °C PBS 80E are shown in Figure 8E.

Generally, all of the polycarbonate scaffolds undergo compression of up to 85% without catastrophic failure, and above 90% with rearrangement of the macroscale struts into a more compressible orientation, before returning to the original geometry (as a function of the material's 4D nature). The same mechanical property trends found in tensile testing were repeatable in compressive loading. Cyclic testing over 100 loading cycles of PTMPCTX in PBS at 37 °C resulted in minimal mechanical behavior change (elastic moduli of 1.1 MPa over the duration of testing; Yield stress = 7.4 MPa vs 5.8 MPa, Ultimate stress = 8.4 MPa *vs* 8.0 MPa respectively, for cycle 1 compared with cycle 100). We postulate that this is a result of the elastic shape memory response, where the shape is fully recovered upon removal of the load as opposed to the thermally driven shape memory response in which the shape is gradually recovered as the material thermally equilibrates. By comparison, the stiffer PNTCTX (compressive elastic moduli of 12.3 MPa) displayed a gradual reduction in recovered strain after each cycle under ambient conditions, decreasing initially by ~25% before stabilizing by cycle 15 at approximately 30% of the original strain; increased delays between compression cycles resulted in further recovery of the material when unloaded.

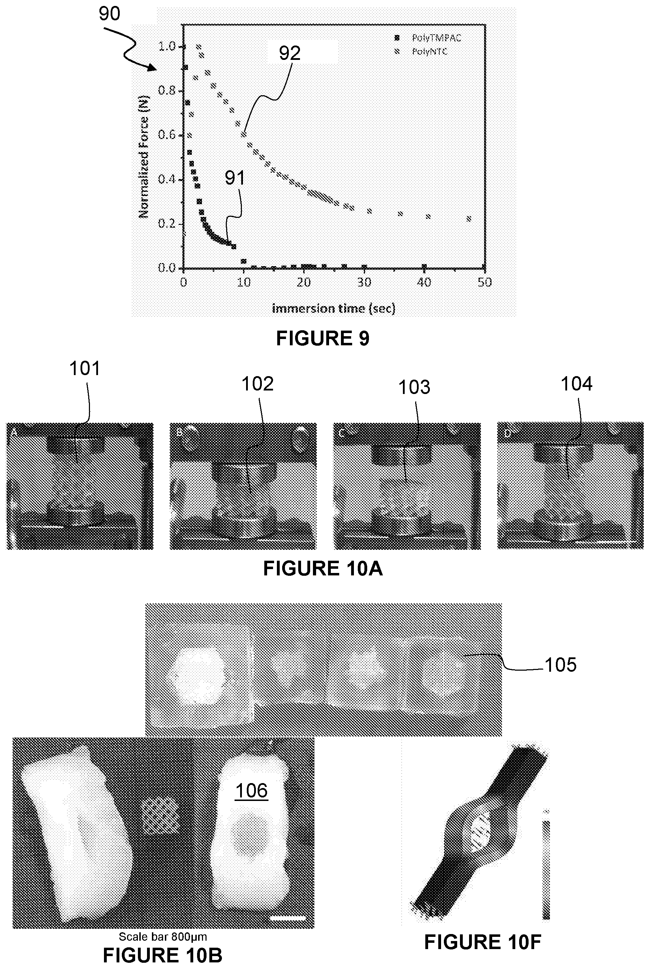

In order to test the mechanical behavior of the 4D scaffolds in a suitable soft tissue-mimicking 3D environment,

alginate hydrogels with tuned temporary crosslinks were selected, owing to their comparable mechanical properties to adipose tissue (elastic moduli of ~60 kPa). Cyclic compressive testing of alginate gels that contain 3D printed scaffolds, similar to the testing of bare scaffolds, was further used to examine the scaffold migration and risk of soft tissue damage that result from the scaffold's presence. After a mock surgical opening using an eye-shaped incision, minimal changes in mechanical behavior were recorded for the compression of gels that contained scaffolds . This indicated that despite the tissue-material mechanical property mismatch that results from their different composition (primarily for PNTCTX scaffolds), the scaffold deforms with the surrounding tissue and remains locked in the void as opposed to non-responsive adipose implants which may migrate *in vivo.* This conclusion is further supported by polymer relaxation studies using immersion DMA, where mechanical properties 90 were measured for polyTMPAC 91 and polyNTC 92 as a function of immersion time in PBS (Figure 9). While the time to the phase transition peak varies with composition, all compositions become fully relaxed at 37 °C in PBS solution. This relaxation behavior is crucial for the designed shape memory response.

**Example 4: 4D Scaffold Behaviour**

**Materials and Methods**

*Shape memory testing:* Shape memory experiments were performed using the same porous scaffolds in compression mode. The samples were equilibrated at 60 °C for 1 h, deformed by ~30% (load dependent deformation) and cooled to -20 °C. Once the sample was isothermal with the cooled chamber, the load was removed and the sample expansion was monitored as a function of force and displacement of the compression clamp as the sample was heated to 60 °C at 10 °C·min-1. Testing was performed in triplicate.

*3D Printing:* Scaffolds based upon previously reported geometries were printed from resins using varied conditions dependent upon composition. Resins were added in 10 mL quantitates to the resin tray, allowing for complete and even coverage of the optical window and the surface of the printing plate. Porous scaffolds were exposed to $\lambda$ = 405 nm light using a custom-built digital light processing unit and printing parameters were individually determined for each resin composition through optimization of irradiance, irradiation time, resulting film thickness, and semi-quantified feature resolution (percentage of theoretical resolution), and were further optimized in the printing vat as necessary. The z-stage transition was set to 100 $\mu$m, and each slice was exposed for 6 s. Print resolution was determined through image analysis (Image J) of the theoretical structure, and pore size analysis using microscopy from the printed structure. The final structures are rinsed with acetone to remove residual resin and photoinhibitor, as denoted by colour removal.

*Degradation Analysis:* Porous scaffolds and non-porous scaffolds were immersed in degradation solution, following previously established protocols for static degradation analysis. For dynamic degradation studies, films were tested using DMA and 5 M NaOH solution at 37 °C, loaded with a 0.1 N pre-load and 10 Hz oscillation. Samples were tested until failure, with the phase ratio and the storage moduli recorded over the course of the study.

For in vivo degradation, samples were removed from subcutaneous tissue and sterilized using EtOH. Tissue was removed and scaffolds were extracted with hexane or methanol over a 48 h period, after which the extracted solutions were concentrated down and dissolved in either CDCl3 or DMSO-d6. Scaffold swelling ratio was determined by:

$$\text{Swelling ratio} = ((m_f - m_i))/m_i$$

where $m_i$ is the original mass of the scaffold (dry) and $m_f$ is the mass of the scaffold after swelling (but blotted dry to remove droplets or excess solvent). The crosslink density, and therefore the remaining mass of the material, was determined by:

$$\text{Gel fraction (\%)} = m_f/m_i$$

where $m_f$ is the final scaffold mass (dry) and $m_i$ is the original scaffold mass (dry).

*Printed Void Filling:* A hexagonal void was produced in Solidworks, and the cross-sectional area was varied to produce irregular voids, one which is sharply irregular and the other possessing rounded edges. The voids were printed and used for studying void filling behavior, using cross-sectional area of the void and the printed scaffold (cube) to determine void filling as a qualitative function of shape.

*Expansion Forces in Alginate Gels:* Alginate was dissolved in water at a concentration of 10 mg·mL-1, to which was added 5 mL of calcium chloride dihydrate (0.1 mg·mL-1). The two components were mixed until gelation, and 10 mL of $H_2O$ was added as the gels were incubated at 37 °C overnight. Gel mechanical properties were matched adipose and

glandular tissue using literature protocol. Gels were cut with an eye-shaped opening, in the same manner as a lumpectomy surgery. Cubic scaffolds were shape fixed at 60% strain and inserted into the opening, where void filling and gel deformation were examined optically using the same cross-sectional analysis described for the "Printed Void Filling" section. The shape fixation behaviour of the scaffold was further examined upon removal of the scaffold from the gel, and the shape recovery efficiency compared with the void filling behaviour, as well as the deformation of the alginate. The thin walled computational models previously described were then examined using determined loading forces and compared with the deformation found in alginate gels. An interior force of 1 N was initially applied uniformly to the interior (cut) surface of the gel in the same manner as the scaffold would be in contact and expand. The force was then scaled until deformation matched experimental results. An FEA analysis is shown in Figure 10F.

**Results**

The carbonate-based materials' shape memory behavior was quantified by DMA in uniaxial tension, optical measurements (samples were compressed to 80% strain and allowed to recover at ambient conditions and at 37 °C in PBS) and comparison of expansion behavior in alginate hydrogels, as well as more rigid acrylate-based 3D printed models, with simplified computational models.

Figure 10A shows the representative shape memory behavior for a printed porous PolyNTC scaffold as it is transitioned from its original geometry (101) to a compressed state under loading (~50% strain, 102), after which it is cooled to 25 °C and will retain its secondary shape after the deformation load is removed (103), and the return to the original geometry upon heating of the sample (104).

The role of NTC content and $T_g$ (both wet and dry) provided direct correlations with strain recovery behavior for compressed polycarbonate scaffolds.

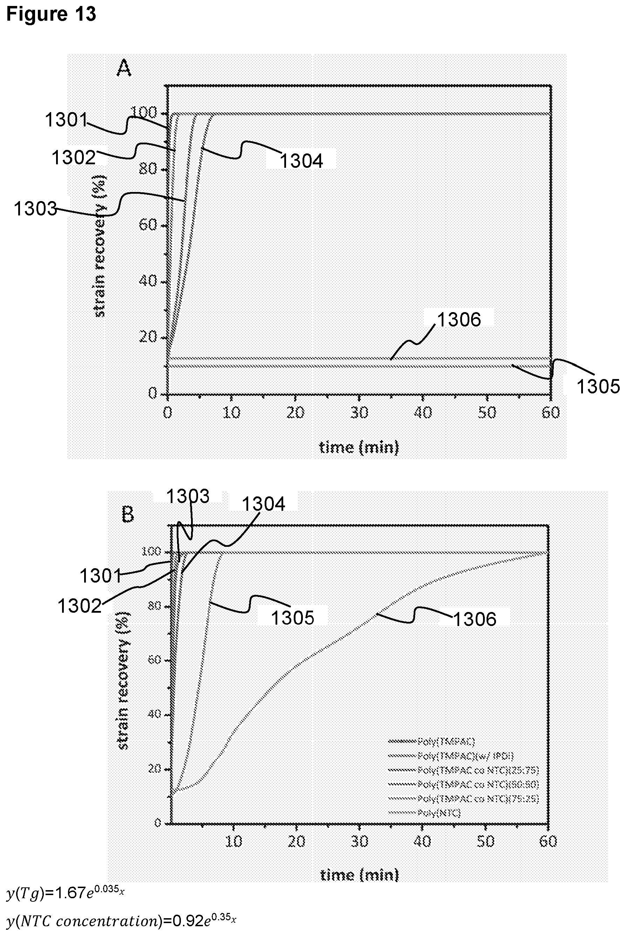

All of the scaffold compositions displayed shape memory behavior (Table 2). Figure 13 shows the strain recovery behavior of printed scaffolds formed from: poly(TMPAC) (1301); poly(TMPAC)(w/IPDI) (1302); poly(TMPAC co NTC) (25:75) (1303); poly(TMPAC co NTC)(50:50) (1304); poly(TMPAC co NTC)(75:25) (1305) and poly(NTC) (1306).

Table 2. Shape memory properties of the printed scaffolds.

| Composition | Strain fixation ($T_g$-20°C), % | Strain fixation ($T_g$), % | Strain recovery ($T_g$ -20°C), % | Strain recovery ($T_g$), % |
|---|---|---|---|---|
| PTMPCTX | 100 | 0 | 51 | 100 |
| P(TM PCTX75-NTCTX25) | 100 | 83 | 0 | 100 |
| P(TMPCTX50-NTCTX50) | 100 | 97 | 0 | 100 |
| P(TMPCTX25-NTCTX75) | 100 | 100 | 0 | 100 |
| PNTCTX | 100 | 100 | 0 | 100 |

Less than 25% NTC content in the starting oligomer decreases strain fixation at room temperature, although all compositions displayed 100% strain fixation and recovery when tested at 20 °C below $T_g$ (tan $\delta$ peak) and 20 °C above $T_g$, respectively. Conversely, increasing NTC content reduced scaffold elasticity, thereby reducing void filling in irregularly shaped rigid voids. The polycarbonate composition altered the strain fixation and the strain recovery kinetics without impacting the stress recovery, which corresponded well with the thermal behavior.

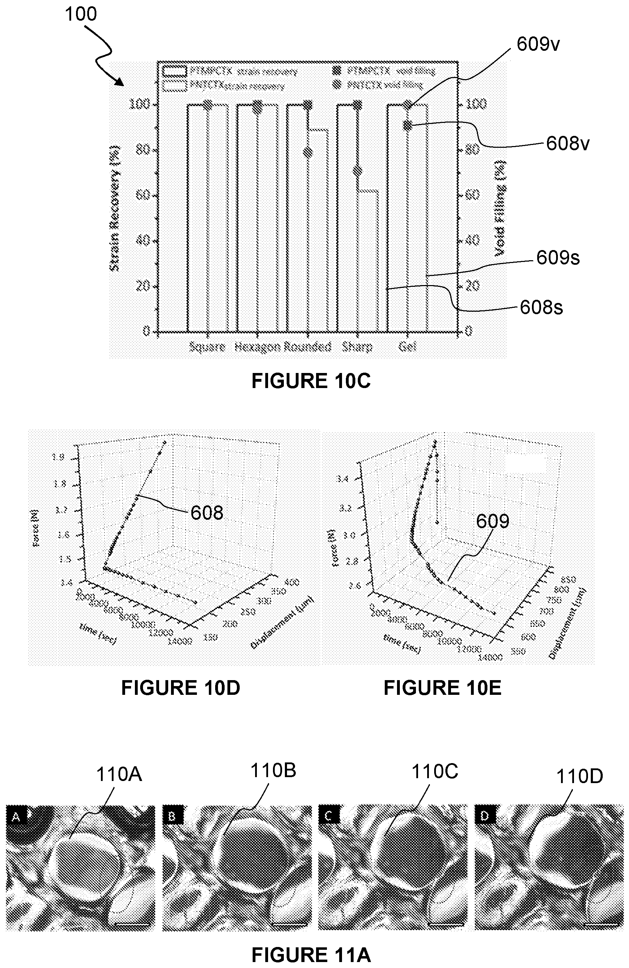

Referring to Figures 10B and 10C, there is shown void filling of various regular and irregular hard (105) and soft (106) voids, produced from 3D printed designs and alginate voids using mock subcutaneous openings. Void filling was measured using cross sectional area after driving full recovery of the scaffold. The scaffolds displayed void filling without deformation of the alginate (PTMPCTX 608v and PNTCTX 609v, 100), and strain recovery (PTMPCTX 608s and PNTCTX 609s, 100) with shape fixation to the void shape even after removal of the scaffold.

Important design features of the printed materials are the expansion forces and the relationship with surrounding soft tissue nerves, as the tissue compression that results from scaffold expansion could result in pain, as well as the need to control material deployment *in vivo.*

Expansion forces of PTMPTCX (608) and PNTCTX (609) using compression kinetic studies under in vitro conditions are shown in Figures 10D and 10E, respectively. PTMPTCX scaffolds underwent rapid shape recovery (100% strain recovery) within 45 s, which distorted the alginate by ~15% (maximum strain) and decreased void filling efficiency to ~90% as a function of scaffold shape. By comparison, the PNTCTX scaffold displayed slower shape recovery. Passive shape recovery at 37 °C required ~50 min for full 100% strain, and had to be stimulated using $H_2O$ at 50 °C (active shape memory) to achieve recovery within 10 mins in the alginate void. PNTCTX scaffolds conform to the soft void with 100% void filling and 90% strain recovery (measured at the center of the scaffold), displaying a low expansion force attributed to

minimal polymer chain reorientation as a result of their high $T_g$. Unlike the PTMPTCX scaffolds, which display only decreasing expansion force with immersion (peak expansion force value of 0.52 N $\pm$ 0.24 N at 37 °C) and an initial relaxation rate of 1.3 mN·s$^{-1}$ (initial 10 mins), PNTCTX displays an increasing tan $\delta$ and storage moduli at 37 °C in PBS, followed by a gradual decrease corresponding with the material's creep response that is indicated by a peak expansion force of 0.71 N $\pm$ 0.19 N (at ~3 mins), and an average relaxation rate of 0.3 mN·s$^{-1}$ (initial 30 mins of immersion). This is ideal behavior because it requires activation of the shape memory response by a surgeon but also enables self-fitting in the soft void. *In vivo,* this will allow for the void-shape fixing without personalization of the scaffold (i.e. a scaffold capable of fitting itself to a variety of soft voids in a similar manner as injectable hydrogels), and over time the ingrowth of clotting factors and tissue would hold the polymer in the final, void-fitted shape. A computational model of a simplistic soft tissue void, using alginate gel mechanical behaviors, revealed maximum deformation similar to what was found experimentally, and indicates the polycarbonates' ability to undergo typical deformations subjected to native tissue during daily life.

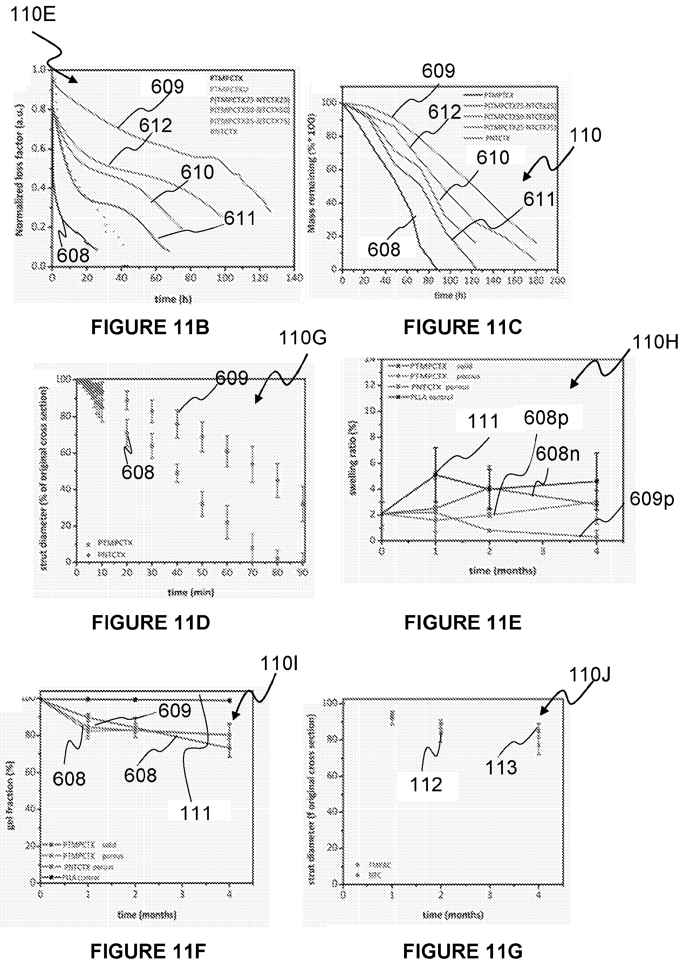

Referring now to Figures 11A to 11D, when exposed to hydrolytic degradation conditions, scaffold surface erosion rates *via* hydrolysis (110A to 110D) could be predicted by thermal transitions (110G, 110H, wherein 111 is a PLLA control); the concentration of base also impacted the acceleration of gravimetric change. Non-porous films were immersed in 5 M NaOH at physiological temperature, and were subjected to 10 $\mu$m deformation at 1 Hz, resulting in material failure behavior (as defined by film erosion and cracking). This trend was similar to what is found using static gravimetric analysis with both films and printed, porous scaffolds, albeit with surface erosion occurring more rapidly as a consequence of the surface deformation caused by mechanical loading. Spectroscopically, as expected, hydrolysis was found to take place at the ester carbonyl in the PETMP as well as along the polycarbonate backbone. All of the materials degraded through a surface erosion behavior that is demonstrated by the gradual reduction in strut cross sectional area in printed scaffolds (110G).

Referring to Figures 11E to 11G, *in vivo* analysis was performed over 4 months in murine subcutaneous implant studies comparing PLLA disks (111), non-porous PTMPTCX disks (608n), and printed, porous disks of PTMPTCX (608p) and PNTCTX (609p) with 500 $\mu$m pores. Material degradation was evaluated using swelling (110H) and gel fraction analysis (110I) post-implantation from subcutaneously implanted samples, and compared with *in vitro* behaviors to approximate mass loss over the implantation period as well as surface erosion rates (110J, wherein 112 is TMPAC and 113 is NTC). Material swelling was found to be statistically unchanged over the course of the 4-month study, with PNTCTX (609) displaying the least swelling compared with the other compositions; PTMPTCX (608) swelling ratio is not affected by the porosity or surface area, which indicates that the minimal swelling that does take place is limited by the crosslink density of the residual network. Regarding the intact thermoset network, all compositions displayed greater than 99% gelation prior to implantation. By month 4, SMPs displayed ca 80% mass remaining, which by extrapolation indicates that total mass loss would most likely occur for the materials within 20 months. Comparatively, the PLLA control materials did not display significant mass loss, which indicates that minimal chain fragmentation is taking place in this same time period. The degradation displayed by the PTMPTCX (608) and PNTCTX (609) would provide sufficient support for more than a year, a seemingly ideal time frame that allows for mature tissue ingrowth before the mechanical support of the scaffolds is sufficiently reduced *via* degradation. Spectroscopic analysis of the implanted samples by FT-IR spectroscopy supports this claim, where minimal shifting of the carbonyl peak indicates less than 30% mass loss has occurred by semi-quantitative analysis of the carbonyl change. This is further supported by $^1$H NMR spectroscopic analysis of extracted samples.

**Example 5: Host-Material Response**

**Materials and Methods**

*Histological Analysis:* At 1-month and 2-month time points, samples were excised from the subcutaneous tissue and fixed with 4% paraformaldehyde for 24 h. After fixation, samples were washed with increasing percentages of ethanol (70% to 100%) for 30 min each, washed thrice with xylene, and embedded in paraffin wax blocks for sectioning. Slices (10-30 $\mu$m thick) were cut using a Leica Biosystems microtome for histological analysis before being stained using hematoxylin and eosin stains or Masson's Trichrome staining using protocols available through Sigma Aldrich. Analysis was performed using light microscopy (Leica, 4X and 10X objectives) and image stitching was performed in ImageJ (NIH, Bethesda, MD). Brightfield images were analyzed and qualitatively assessed for general inflammation compared to PLLA control samples. Samples were also analyzed for a number of inflammatory cells utilizing a modified scoring system designed by the International Organization for Standardization (ISO 10993-6 Annex E). Scoring was based on a scale from 0-4 (0 = none; 1 = Rare, 1-5 Minimal; 2 = 5-10, Mild; 3 = Heavy Infiltrate, Moderate; 4 = Packed, Severe).

*Statistical Analysis:* In all Examples described herein, statistical analysis of results was performed using a standard one-way Student's t-test, with probabilities of 0.01 and 0.05 used to assess the probability of differences between compositional behaviors.

**Results**

Histopathological analysis further indicated the promise of these materials for tissue engineering applications.

Figure 12 shows representative histological images from PLLA control materials at 1 month (A) and 4 months (E) compared with PTMPTCX films at the same times (B, F). Masson's Trichrome (C, D) and H&E (H, I) images of PTMPTCX (C, H) and PNTCTX (D, I) printed scaffolds after 4 months, respectively, with corresponding histological scoring and assessment.

H&E and Trichrome stains revealed the presence of adipocyte infiltration by the 1-month time point in the porous prints, with minimal lobule formation at this time. However, by 2 months distinct lobules were seen within the pores of the scaffolds as well as on the periphery at the material-tissue original interface which indicates restoration of normal tissue as opposed to damaged or scarred tissue. For non-porous polycarbonate-derived material disks, lobules were found within 100 μm of the material surface. Adipocyte shape *in vivo* further reflects the positive response to the surface, as the characteristic round morphology is found within both the lobules as well as individually. Our results indicate that nearly 40% of the infiltrated tissue is represented by adipocyte lobules, with fibroblasts representing another majority of the tissue; PLLA did not display this type of integration over the same time period. Capsule formation around all examined implants was less than 200 μm thick, well below the 500 μm threshold used for biocompatibility in other studies. Importantly, the capsule formation was reduced with increased surface area; the porous implants displayed approximately half the capsule thickness (~50 μm) as solid polycarbonates, which suggests that there may be a benefit to the 3D structure for tissue engineering implants; PLLA displayed ~120 μm capsules. Macrophage presence (Table 3) was found to be indicative of healing rather than severe inflammatory response, and the presence of macrophages have been linked to healthy function in adipose tissue, supported by fibroblast presence.

Vascular bud formation and vascularization occurred by 2 months, with several small, mature vessels found at 4 months in the surrounding tissue but no additional budding. Vascular budding allows for healing to occur, and then ideally will be reduced to match the original tissue as seen here, as adipose tissue is typically not heavily vascularized. One of the main failures in contemporary clinical techniques for restoring soft tissue, such as in adipose repair using autologous fat transplantation, is the 40-60% loss of graft volume as a result of poor graft vascularization post-implantation, and aspirated adipocytes are easily damaged by the mechanical force of the procedure, ultimately leading to cyst and localized necrosis that causes immune response and loss of the graft. No calcification was found in the implants, nor was necrosis.

Table 3. Pathological scoring of the polycarbonate implants (out of 5).

| Time | Location | Sample | Adipocytes | Neutrophils | Lymphocytes | Plasma Cells | Mononuclear Macrophage | Multinucleated Foreign Giant Cells | Platelets | Necrosis | Neovascularization |
|---|---|---|---|---|---|---|---|---|---|---|---|
| 1 Month | Exterior | Solid PTMPCTX | 0.9 ± 0.4 | 0.0 ± 0.0 | 0.0 ± 0.0 | 0.1 ± 0.1 | 1.7 ± 1.2 | 0.0 ± 0.0 | --- | 0.0 ± 0.0 | --- |
| 1 Month | Exterior | Porous PTMPCTX | 1.7 ± 0.9 | 0.0 ± 0.0 | 0.0 ± 0.0 | 0.0 ± 0.0 | 2.6 ± 0.7 | 0.0 ± 0.0 | --- | 0.0 ± 0.0 | --- |
| 1 Month | Exterior | Porous PNTCX | 2.2 ± 0.7 | 0.0 ± 0.1 | 0.0 ± 0.0 | 0.0 ± 0.0 | 2.2 ± 0.7 | 0.0 ± 0.0 | --- | 0.0 ± 0.0 | --- |
| 1 Month | Interior | Solid PTMPCTX | --- | --- | --- | --- | --- | --- | --- | --- | --- |
| 1 Month | Interior | Porous PTMPCTX | 2.1 ± 0.6 | 0.0 ± 0.0 | 0.0 ± 0.0 | 0.0 ± 0.0 | 2.6 ± 0.9 | 0.0 ± 0.0 | 2.1 ± 1.1 | 0.0 ± 0.0 | 0.3 ± 0.5 |
| 1 Month | Interior | Porous PNTCX | 3.0 ± 1.2 | 0.0 ± 0.0 | 0.0 ± 0.0 | 0.0 ± 0.0 | 2.1 ± 0.8 | 0.0 ± 0.0 | 2.7 ± 1.2 | 0.0 ± 0.0 | 0.3 ± 0.4 |
| 2 Month | Exterior | Solid PTMPCTX | 1.5 ± 0.8 | 0.0 ± 0.0 | 0.0 ± 0.0 | 0.1 ± 0.2 | 1.4 ± 0.9 | 0.0 ± 0.0 | --- | 0.0 ± 0.0 | --- |
| 2 Month | Exterior | Porous PTMPCTX | 3.0 ± 1.1 | 0.0 ± 0.0 | 0.0 ± 0.0 | 0.0 ± 0.0 | 2.2 ± 1.0 | 0.0 ± 0.0 | --- | 0.0 ± 0.0 | --- |
| 2 Month | Exterior | Porous PNTCX | 3.2 ± 1.3 | 0.0 ± 0.1 | 0.0 ± 0.0 | 0.0 ± 0.0 | 3.1 ± 1.3 | 0.0 ± 0.0 | --- | 0.0 ± 0.0 | --- |
| 2 Month | Interior | Solid PTMPCTX | --- | --- | --- | --- | --- | --- | --- | --- | --- |
| 2 Month | Interior | Porous PTMPCTX | 3.0 ± 1.1 | 0.0 ± 0.0 | 0.0 ± 0.0 | 0.0 ± 0.0 | 2.5 ± 1.1 | 0.0 ± 0.0 | 2.8 ± 1.3 | 0.0 ± 0.0 | 1.1 ± 0.6 |
| 2 Month | Interior | Porous PNTCX | 3.2 ± 1.3 | 0.0 ± 0.0 | 0.0 ± 0.0 | 0.0 ± 0.0 | 3.1 ± 1.3 | 0.0 ± 0.0 | 2.6 ± 1.1 | 0.0 ± 0.0 | 0.7 ± 0.6 |
| 4 Month | Exterior | Solid PTMPCTX | 2.1 ± 0.9 | 0.0 ± 0.1 | 0.0 ± 0.0 | 0.1 ± 0.3 | 2.1 ± 1.2 | 0.0 ± 0.0 | --- | 0.0 ± 0.0 | --- |
| 4 Month | Exterior | Porous PTMPCTX | 3.6 ± 0.8 | 0.0 ± 0.2 | 0.0 ± 0.0 | 0.0 ± 0.0 | 3.1 ± 1.5 | 0.0 ± 0.0 | --- | 0.0 ± 0.0 | --- |
| 4 Month | Exterior | Porous PNTCX | 3.3 ± 0.9 | 0.0 ± 0.3 | 0.0 ± 0.0 | 0.0 ± 0.0 | 3.0 ± 1.6 | 0.0 ± 0.0 | --- | 0.0 ± 0.0 | --- |
| 4 Month | Interior | Solid PTMPCTX | --- | --- | --- | --- | --- | --- | --- | --- | --- |
| 4 Month | Interior | Porous PTMPCTX | 3.2 ± 0.9 | 0.0 ± 0.0 | 0.0 ± 0.0 | 0.0 ± 0.0 | 2.9 ± 1.4 | 0.0 ± 0.0 | 1.1 ± 0.8 | 0.0 ± 0.0 | 2.1 ± 1.1 |
| 4 Month | Interior | Porous PNTCX | 3.3 ± 0.7 | 0.0 ± 0.0 | 0.0 ± 0.0 | 0.0 ± 0.0 | 3.1 ± 1.1 | 0.0 ± 0.0 | 1.0 ± 0.9 | 0.0 ± 0.0 | 2.4 ± 1.2 |

## Claims

1. A void occlusion implant (10, 44) for inserting into a void (43) in a body tissue, the implant (10, 44) comprising a polymeric material which is capable of transitioning from a compressed state (44a) to an expanded state (44b) upon exposure to a stimulus, wherein in the expanded state (44b) the implant (10, 44) is capable of assuming the size and shape of the void (43) and wherein the implant (10, 44) exhibits a peak expansion force of 0.1 to 2 N at 37°C;

wherein the polymeric material is formed from a resin composition comprising a prepolymer and a cross-linker; wherein the prepolymer is poly(TMPAC) ((5-[(allyloxy)methyl]-5-ethyl-1,3-dioxan-2-one)), poly(NTC) ((9-(5-norbornen-2-yl)-2,4,8,10-tetraoxa-3-spiro[5.5]undecanone)) or poly(TMPAC-co-NTC); and
wherein the cross-linker comprises one or more thiol moieties such that the polymeric material is a polyTMPAC-derived thioether crosslinked resin (PTMPCTX), a polyNTC-derived thioether crosslinked resin (PNTCTX) or a poly(TMPAC-co-NTC) thioether crosslinked resin (P(TMPCTX-NTCTX)).

**2.** The void occlusion implant (10, 44) of claim 1, wherein the implant is a post-lumpectomy implant (44).

**3.** The void occlusion implant (10, 44) of claim 1 or claim 2, wherein the implant (10, 44) is 3D printed.

**4.** The void occlusion implant (10, 44) of any one of claims 1 to 3, wherein the resin composition further comprises one or more diluents, wherein either or both of the prepolymer and the at least one diluent comprises at least one O=C-N linkage, preferably a urethane linkage.

**5.** The void occlusion implant (10, 44) of claim 4, wherein the ratio of TMPAC (5-[(allyloxy)methyl]-5-ethyl-1,3-dioxan-2-one) to NTC (9-(5-norbornen-2-yl)-2,4,8,10-tetraoxa-3- spiro[5.5]undecanone) monomers in the prepolymer is from 95:5 to 5:95.

**6.** The void occlusion implant (10, 44) of any preceding claim, wherein the implant (10, 44) has *in vivo* life of no more than 36 months.

**7.** The void occlusion implant (10, 44) of any preceding claim, wherein the polymeric material comprises an imaging agent, optionally wherein the imaging agent comprises a radiopaque material, a radiotracer, or a fluorescent dye and/or wherein the polymeric material comprises a biologically active agent, optionally wherein the biologically active agent is selected from an antimicrobial, an anti-inflammatory agent, a growth factor or an anti-cancer agent.

**8.** The void occlusion implant (10, 44) of any preceding claim, wherein the implant (10, 44) is in the form of a foam or mesh having a pore size of from 50 to 2000 μm.

**9.** A kit for reconstruction of tissue following a surgical procedure, the kit comprising at least one void occlusion implant (10, 44) according to any one of claims 1 to 8, and instructions for use.

**10.** The kit of claim 9, wherein the kit comprises at least two void occlusion implants (10, 44) which differ from each other in at least their size, shape, material or mechanical properties.

**11.** The kit of claim 9 or claim 10, wherein the kit further comprises at least one of:

- an instrument for inserting the void occlusion implant (10, 44) into the void (43);
- apparatus for compressing the void occlusion implant (10, 44) prior to insertion; and/or
- a stimulating device or reagent for causing the void occlusion implant (10, 44) to transition from a compression (44a) to an expanded state (44b).

**12.** A method of manufacturing a void occlusion implant (10, 44), the method comprising

(i) providing a resin composition comprising a prepolymer and optionally one or more diluent(s), wherein the prepolymer is poly(TMPAC) ((5-[(allyloxy)methyl]-5-ethyl-1,3-dioxan-2-one)), poly(NTC) ((9-(5-norbornen-2-yl)-2,4,8,10-tetraoxa-3-spiro[5.5]undecanone)) or poly(TMPAC-co-NTC);
(ii) shaping the resin composition into a desired size and shape of the implant (10, 44); and
(iii) cross-linking the prepolymer with a cross-linker comprising one or more thiol moieties, thereby forming an implant (10, 44) having a peak expansion force of 0.1 to 2 N at 37°C when transitioning from a first compressed state (44a) to a second uncompressed state (44b), wherein the implant comprises polyTMPAC-derived thioether crosslinked resin (PTMPCTX), a polyNTC-derived thioether crosslinked resin (PNTCTX) or a poly(TMPAC-co-NTC) thioether crosslinked resin (P(TMPCTX-NTCTX)).

**13.** The method of claim 12, wherein steps (ii) and (iii) are carried out simultaneously, optionally by 3D printing (e.g. stereolithography).

**14.** The method of claim 12 or 13, wherein the method further comprises modifying the void occlusion implant (10, 44) by turning, milling, sanding, filing, cutting, drilling and/or compressing the implant (10, 44), wherein compressing the void occlusion implant (10, 44) may comprise:

- heating the implant (10, 44) to a temperature greater than the glass transition temperature of the polymeric material;
- compressing the implant (10, 44); and
- fixing the implant (10, 44) in the compressed form, optionally by cooling.

**15.** The method of any one of claims 12 to 14, wherein the method further comprises determining the dimensions of the void (43), and manufacturing a void occlusion implant (10, 44) having a desired size and shape based on the determined dimensions of the void (43) and/or wherein the method further comprises adding a biologically active agent and/or an imaging agent to the resin composition and/or to the polymeric material.

**Patentansprüche**

**1.** Hohlraumverschlussimplantat (10, 44) zum Einsetzen in einen Hohlraum (43) in einem Körpergewebe, wobei das Implantat (10, 44) ein polymeres Material umfasst, das in der Lage ist, bei Exposition gegenüber einem Reiz von einem komprimierten Zustand (44a) in einen expandierten Zustand (44b) überzugehen, wobei das Implantat (10, 44) in dem expandierten Zustand (44b) in der Lage ist, die Größe und Form des Hohlraums (43) anzunehmen, und wobei das Implantat (10, 44) eine maximale Expansionskraft von 0,1 bis 2 N bei 37 °C zeigt;

wobei das polymere Material aus einer Harzzusammensetzung ausgebildet ist, die ein Vorpolymer und einen Vernetzer umfasst;
wobei das Vorpolymer Poly(TMPAC) ((5-[(Allyloxy)methyl]-5-ethyl-1,3-dioxan-2-on)), Poly(NTC) ((9-(5-Norbornen-2-yl)-2,4,8,10-tetraoxa-3-spiro[5.5]undecanon)) oder Poly(TMPAC-co-NTC) ist; und
wobei der Vernetzer eine oder mehrere Thioleinheiten umfasst, sodass das polymere Material ein von Poly-TMPAC abgeleitetes vernetztes Thioetherharz (PTMPCTX), ein von PolyNTC abgeleitetes vernetztes Thioetherharz (PNTCTX) oder ein vernetztes Poly(TMPAC-co-NTC)-Thioetherharz (P(TMPCTX-NTCTX)) ist.

**2.** Hohlraumverschlussimplantat (10, 44) nach Anspruch 1, wobei das Implantat ein Post-Lumpektomie-Implantat (44) ist.

**3.** Hohlraumverschlussimplantat (10, 44) nach Anspruch 1 oder Anspruch 2, wobei das Implantat (10, 44) 3D-gedruckt ist.

**4.** Hohlraumverschlussimplantat (10, 44) nach einem der Ansprüche 1 bis 3, wobei die Harzzusammensetzung ferner ein oder mehrere Verdünnungsmittel umfasst, wobei eines oder beide von dem Vorpolymer und dem mindestens einen Verdünnungsmittel mindestens eine O= C-N-Verknüpfung, vorzugsweise eine Urethan-Verknüpfung, umfassen.

**5.** Hohlraumverschlussimplantat (10, 44) nach Anspruch 4, wobei das Verhältnis von TMPAC(5-[(Allyloxy)methyl]-5-ethyl-1,3-dioxan-2-on)- zu NTC(9-(5-Norbornen-2-yl)-2,4,8,10-tetraoxa-3-spiro[5.5]undecanon)-Monomeren in dem Vorpolymer 95:5 bis 5:95 beträgt.

**6.** Hohlraumverschlussimplantat (10, 44) nach einem vorhergehenden Anspruch, wobei das Implantat (10, 44) eine In-vivo-Lebensdauer von nicht mehr als 36 Monaten aufweist.

**7.** Hohlraumverschlussimplantat (10, 44) nach einem vorhergehenden Anspruch, wobei das polymere Material ein bildgebendes Mittel umfasst, wobei das bildgebende Mittel optional ein strahlenundurchlässiges Material, einen Radiotracer oder einen Fluoreszenzfarbstoff umfasst und/oder wobei das polymere Material ein biologisch aktives Mittel umfasst, wobei das biologisch aktive Mittel optional aus einem antimikrobiellen Mittel, einem entzündungshemmenden Mittel, einem Wachstumsfaktor oder einem Antikrebsmittel ausgewählt ist.

**8.** Hohlraumverschlussimplantat (10, 44) nach einem vorhergehenden Anspruch, wobei das Implantat (10, 44) in Form eines Schaums oder Netzes mit einer Porengröße von 50 bis 2000 μm vorliegt.

9. Kit zur Rekonstruktion von Gewebe nach einem chirurgischen Eingriff, wobei das Kit mindestens ein Hohlraumverschlussimplantat (10, 44) gemäß einem der Ansprüche 1 bis 8 und eine Gebrauchsanweisung umfasst.

10. Kit nach Anspruch 9, wobei das Kit mindestens zwei Hohlraumverschlussimplantate (10, 44) umfasst, die sich mindestens in ihrer Größe, Form, ihrem Material oder ihren mechanischen Eigenschaften voneinander unterscheiden.

11. Kit nach Anspruch 9 oder Anspruch 10, wobei das Kit ferner mindestens eines von Folgenden umfasst:

- ein Instrument zum Einsetzen des Hohlraumverschlussimplantats (10, 44) in den Hohlraum (43);
- Einrichtung zum Komprimieren des Hohlraumverschlussimplantats (10, 44) vor dem Einsetzen; und/oder
- eine Stimulationsvorrichtung oder ein Reagenz, um zu bewirken, dass das Hohlraumverschlussimplantat (10, 44) von einer Kompression (44a) in einen expandierten Zustand (44b) übergeht.

12. Verfahren zum Herstellen eines Hohlraumverschlussimplantats (10, 44), wobei das Verfahren Folgendes umfasst:

(i) Bereitstellen einer Harzzusammensetzung, die ein Vorpolymer und optional ein oder mehrere Verdünnungsmittel umfasst, wobei das Vorpolymer Poly(TMPAC) ((5-[(Allyloxy)methyl]-5-ethyl-1,3-dioxan-2-on)), Poly(NTC) ((9-(5-Norbornen-2-yl)-2,4,8,10-tetraoxa-3-spiro[5.5]undecanon)) oder Poly(TMPAC-co-NTC) ist;
(ii) Formen der Harzzusammensetzung in eine gewünschte Größe und Form des Implantats (10, 44); und
(iii) Vernetzen des Vorpolymers mit einem Vernetzer, der eine oder mehrere Thioleinheiten umfasst, wodurch ein Implantat (10, 44) mit einer maximalen Expansionskraft von 0,1 bis 2 N bei 37 °C beim Übergehen von einem ersten komprimierten Zustand (44a) in einen zweiten unkomprimierten Zustand (44b) ausgebildet wird, wobei das Implantat ein von PolyTMPAC abgeleitetes vernetztes Thioetherharz (PTMPCTX), ein von PolyNTC abgeleitetes vernetztes Thioetherharz (PNTCTX) oder ein vernetztes Poly(TMPAC-co-NTC)-Thioetherharz (P(TMPCTX-NTCTX)) umfasst.

13. Verfahren nach Anspruch 12, wobei die Schritte (ii) und (iii) gleichzeitig ausgeführt werden, optional durch 3D-Drucken (z. B. Stereolithographie).

14. Verfahren nach Anspruch 12 oder 13, wobei das Verfahren ferner Modifizieren des Hohlraumverschlussimplantats (10, 44) durch Drehen, Fräsen, Schleifen, Feilen, Schneiden, Bohren und/oder Komprimieren des Implantats (10, 44) umfasst, wobei Komprimieren des Hohlraumverschlussimplantats (10, 44) Folgendes umfassen kann:

- Erwärmen des Implantats (10, 44) auf eine Temperatur, die höher als die Glasübergangstemperatur des polymeren Materials ist;
- Komprimieren des Implantats (10, 44); und
- Fixieren des Implantats (10, 44) in der komprimierten Form, optional durch Kühlen.

15. Verfahren nach einem der Ansprüche 12 bis 14, wobei das Verfahren ferner Bestimmen der Abmessungen des Hohlraums (43) und Herstellen eines Hohlraumverschlussimplantats (10, 44) mit einer gewünschten Größe und Form basierend auf den bestimmten Abmessungen des Hohlraums (43) umfasst und/oder wobei das Verfahren ferner Hinzufügen eines biologisch aktiven Mittels und/oder eines bildgebenden Mittels zu der Harzzusammensetzung und/oder zu dem polymeren Material umfasst.

## Revendications

1. Implant d’occlusion de vide (10, 44) destiné à être inséré dans un vide (43) dans un tissu corporel, l’implant (10, 44) comprenant un matériau polymère qui est capable de passer d’un état comprimé (44a) à un état expansé (44b) lors de l’exposition à un stimulus, à l’état expansé (44b) ledit implant (10, 44) étant capable de prendre la taille et la forme du vide (43) et ledit implant (10, 44) présentant une force d’expansion maximale de 0,1 à 2 N à 37°C ;

ledit matériau polymère étant formé à partir d’une composition de résine comprenant un prépolymère et un agent de réticulation ;
ledit prépolymère étant le poly(TMPAC) ((5-[(allyloxy)méthyl]-5-éthyl-1,3-dioxan-2-one)), le poly(NTC) ((9-(5-norbornén-2-yl)-2,4,8,10-tétraoxa-3-spiro[5.5]undécanone)) ou le poly(TMPAC-co-NTC) ; et
ledit agent de réticulation comprenant un ou plusieurs groupements thiol de sorte que le matériau polymère soit

une résine réticulée de thioéther dérivée de polyTMPAC (PTMPCTX), une résine réticulée de thioéther dérivée de polyNTC (PNTCTX) ou une résine réticulée de poly(TMPAC-co-NTC) thioéther (P(TMPCTX-NTCTX)).

**2.** Implant d'occlusion de vide (10, 44) selon la revendication 1, ledit implant étant un implant de post-lumpectomie (44).

**3.** Implant d'occlusion de vide (10, 44) selon la revendication 1 ou la revendication 2, ledit implant (10, 44) étant imprimé en 3D.

**4.** Implant d'occlusion de vide (10, 44) selon l'une quelconque des revendications 1 à 3, ladite composition de résine comprenant en outre un ou plusieurs diluants, l'un ou l'autre ou les deux parmi le prépolymère et ledit au moins un diluant comprenant au moins une liaison O=C-N, de préférence une liaison uréthane.

**5.** Implant d'occlusion de vide (10, 44) selon la revendication 4, ledit rapport des monomères TMPAC (5-[(allyloxy) méthyl]-5-éthyl-1,3-dioxan-2-one) à la NTC (9-(5-norbornén-2-yl)-2,4,8,10-tétraoxa-3-spiro[5.5]undécanone) dans le prépolymère valant de 95:5 à 5:95.

**6.** Implant d'occlusion de vide (10, 44) selon l'une quelconque des revendications précédentes, ledit implant (10, 44) présentant une durée de vie *in vivo* ne dépassant pas 36 mois.

**7.** Implant d'occlusion de vide (10, 44) selon l'une quelconque revendication précédente, ledit matériau polymère comprenant un agent d'imagerie, éventuellement ledit agent d'imagerie comprenant un matériau radio-opaque, un radiotraceur ou un colorant fluorescent et/ou ledit matériau polymère comprenant un agent biologiquement actif, éventuellement ledit agent biologiquement actif étant choisi parmi un agent antimicrobien, un agent anti-inflammatoire, un facteur de croissance ou un agent anticancéreux.

**8.** Implant d'occlusion de vide (10, 44) selon l'une quelconque des revendications précédentes, ledit implant (10, 44) se présentant sous la forme d'une mousse ou d'un treillis présentant une taille de pore de 50 à 2000 μm.

**9.** Kit destiné à la reconstruction de tissu après une intervention chirurgicale, le kit comprenant au moins un implant d'occlusion de vide (10, 44) selon l'une quelconque des revendications 1 à 8, et des instructions d'utilisation.

**10.** Kit selon la revendication 9, ledit kit comprenant au moins deux implants d'occlusion de vide (10, 44) qui diffèrent l'un de l'autre par au moins leur taille, leur forme, leur matériau ou leurs propriétés mécaniques.

**11.** Kit selon la revendication 9 ou la revendication 10, ledit kit comprenant en outre au moins un élément parmi :

- un instrument destiné à l'insertion de l'implant d'occlusion de vide (10, 44) dans le vide (43) ;
- un appareil destiné à la compression de l'implant d'occlusion de vide (10, 44) avant l'insertion ; et/ou
- un dispositif de stimulation ou un réactif pour amener l'implant d'occlusion de vide (10, 44) à passer d'un état comprimé (44a) à un état expansé (44b).

**12.** Procédé de fabrication d'un implant d'occlusion de vide (10, 44), le procédé comprenant

(i) la fourniture d'une composition de résine comprenant un prépolymère et éventuellement un ou plusieurs diluants, ledit prépolymère étant le poly(TMPAC) ((5-[(allyloxy)méthyl]-5-éthyl-1,3-dioxan-2-one)), le poly(NTC) ((9-(5-norbornén-2-yl)-2,4,8,10-tétraoxa-3-spiro[5.5]undécanone)) ou le poly(TMPAC-co-NTC) ;
(ii) la mise en forme de la composition de résine selon une taille et une forme souhaitées de l'implant (10, 44) ; et
(iii) la réticulation du prépolymère avec un agent de réticulation comprenant un ou plusieurs groupements thiol, formant ainsi un implant (10, 44) présentant une force d'expansion maximale de 0,1 à 2 N à 37°C lors du passage d'un premier état comprimé (44a) à un second état expansé (44b), ledit implant comprenant une résine réticulée de thioéther dérivée de polyTMPAC (PTMPCTX), une résine réticulée de thioéther dérivée de polyNTC (PNTCTX) ou une résine réticulée de poly(TMPAC-co-NTC) thioéther (P(TMPCTX- NTCTX)).

**13.** Procédé selon la revendication 12, lesdites étapes (ii) et (iii) étant effectuées simultanément, éventuellement par impression 3D (par exemple, stéréolithographie).

**14.** Procédé selon la revendication 12 ou 13, ledit procédé comprenant en outre la modification de l'implant d'occlusion de vide (10, 44) par tournage, fraisage, ponçage, limage, découpe, perçage et/ou compression de l'implant (10, 44),

ladite compression de l'implant d'occlusion de vide (10, 44) pouvant comprendre :

- le chauffage de l'implant (10, 44) à une température supérieure à la température de transition vitreuse du matériau polymère ;
- la compression de l'implant (10, 44) ; et
- la fixation de l'implant (10, 44) sous la forme comprimée, éventuellement par refroidissement.

**15.** Procédé selon l'une quelconque des revendications 12 à 14, ledit procédé comprenant en outre la détermination des dimensions du vide (43), et la fabrication d'un implant d'occlusion de vide (10, 44) présentant une taille et une forme souhaitées sur la base des dimensions déterminées du vide (43) et/ou ledit procédé comprenant en outre l'ajout d'un agent biologiquement actif et/ou d'un agent d'imagerie à la composition de résine et/ou au matériau polymère.

10

FIGURE 1

A
B
C
D
E
F
H
I

FIGURE 12

20
21
(a)
COClEt
NEt3
0 °C
22
23
24
H2O, HCl
(b)
25
(c)
COClEt
NEt3
0 °C
26
DBU, H2O
(d)
DBU=
23
27
(e)
O=C=N-R-N=C=O
28
29
O=C=N-R-N=C=O =
28

**FIGURE 2**

30A
FG
31
FG'
32

FIGURE 3A

31
32
30B
X-ray density (gray density)
0
10
20
30
40
50

FIGURE 3B

30C
31
RSH, hv
FG
33
FG'
S
R

FIGURE 3C

40
40A
40B
40C
42
41
41
43
44a
44b

FIGURE 4

52
52
51
51
50A
50B
50C
50A2
50B2

**FIGURE 5**

60A
Inhibitor
Irgacure 819
405 nm
603
602
601
normalized absorbance (a.u.)
350
360
370
380
390
400
410
420
430
440
450
wavelength (nm)

**FIGURE 6A**

612
60B
611
610
609
608
tan δ (E''/E')
1.0
0.8
0.6
0.4
0.2
0.0
0
20
40
60
80
100
120
140
time (s)


**FIGURE 6B**

606
60C
604
605
607
conversion (%)
100
80
60
40
20
0
0
50
100
150
200
250
300
time (sec)
PolyTMPAC-PETMP
PolyNTC-PETMP
Reactive Diluent-PETMP
Poly(TMPAC-IPDI)-PETMP


**FIGURE 6C**

63

60D
608
612
610
611
Storage Modulus (E') (MPa)
0
20
40
60
80
100
120
140
Time (s)


**FIGURE 6D**

60E
-0.14
-0.12
-0.10
-0.08
-0.06
-0.04
-0.02
0.00
Shrinkage (mm/mm)
0
20
40
60
80
100
120
140
160
Time (s)
611
609
608
612

FIGURE 6E

60F
1E12
1E11
1E10
1E9
1E8
1E7
1000000
100000
10000
1000
100
10
1
viscosity (mPa*s)
0.5%
0.1%
no initiator
0
5
10
15
20
25
30
35
time (s)
613
614
615

FIGURE 6F

60G
800
700
600
500
400
300
200
100
0
viscosity (mPa*s)
0
20
40
60
80
100
solvent (wt %)
616

FIGURE 6G

60H
617
viscosity (mPa*s)
Photoinitiator concentration (wt %)
0.00
0.02
0.04
0.06
0.08
0.10

FIGURE 6H

60I
618
619

FIGURE 6I

70A
701
702
703
704
A
B
C
D

FIGURES 7A

70B
706
705
707
E
F

FIGURE 7B

70C
G
710
709
708
708
710
SMOOTH SIDE
TOP
STEPPED SIDE
709

FIGURE 7C

711
713
712
70D
H
TOP
MIDDLE
BOTTOM

FIGURE 7D

80A
81
82
glass transition temperature (°C)
composition (% NTC)
609
612
80B
610
611
608
PNTCTX
PTMPCTX
stress (MPa)
strain (%)
80C
84
85
cycle 1
cycle 100
stress (MPa)
strain (%)
80E
609
610
608
energy dissipation (J)
number of cycles
PTMPCTX
PNTCTX
80D
86
87
88

FIGURE 8A

FIGURE 8B

FIGURE 8C

FIGURE 8E

FIGURE 8D

90
92
91
PolyTMPAC
PolyNTC
Normalized Force (N)
immersion time (sec)
0.0
0.2
0.4
0.6
0.8
1.0
0
10
20
30
40
50

FIGURE 9

101
102
103
104
A
B
C
D

FIGURE 10A

105
106
Scale bar 800μm

FIGURE 10B

FIGURE 10F

100
609v
608v
609s
608s
PTMPCTX strain recovery
PNTCTX strain recovery
PTMPCTX void filling
PNTCTX void filling
Strain Recovery [%]
Void Filling [%]
Square
Hexagon
Rounded
Sharp
Gel

FIGURE 10C

608
609
Force [N]
time [sec]
Displacement [µm]

FIGURE 10D

FIGURE 10E

110A
110B
110C
110D
A
B
C
D

FIGURE 11A

110E
609
612
610
611
608
time (h)
FIGURE 11B
609
612
110
610
611
608
time (h)
FIGURE 11C
110G
609
608
time (min)
FIGURE 11D
110H
111
608p
608n
609p
time (months)
FIGURE 11E
110I
609
608
608
111
time (months)
FIGURE 11F
110J
112
113
time (months)
FIGURE 11G

## Figure 13

A
1301
1302
1303
1304
1305
1306
strain recovery (%)
time (min)
0
20
40
60
80
100
0
10
20
30
40
50
60

B
1301
1302
1303
1304
1305
1306
strain recovery (%)
time (min)
0
20
40
60
80
100
10
20
30
40
50
60
Poly(TMPAC)
Poly(TMPAC)(w/ IPDI)
Poly(TMPAC co NTC)(25:75)
Poly(TMPAC co NTC)(50:50)
Poly(TMPAC co NTC)(75:25)
Poly(NTC)

$y(Tg)=1.67e^{0.035x}$

$y(NTC\ concentration)=0.92e^{0.35x}$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2019038812 A1


### Non-patent literature cited in the description


- **IA BARKER**. *Biomaterials Science*, 2014, vol. 2, 472-475
- **Y HE**. *Reactive and Functional Polymers*, February 2011, vol. 71 (2), 175-186